# EUROPEAN PATENT SPECIFICATION

(11) **EP 1 906 972 B1**
(45) Date of publication and mention of the grant of the patent: **11.09.2013**
(21) Application number: 06787646.6
(22) Date of filing: 19.07.2006
(51) Int. Cl.: A61K 45/06, A61K 31/661, A61K 31/665, A61K 31/66, A61K 31/685, A61P 1/12

(54) **LPA2 receptor agonist for treating diarrhea**
LPA2-Rezeptoragonist zur Behandlung von Durchfall
Agoniste du recepteur LPA2 pour le traitement de la diarrhée

(30) Priority: 19.07.2005 US 700489 P
(43) Date of publication of application: 09.04.2008
(73) Proprietor: The University of Tennessee Research Foundation, Knoxville, TN 37996-1527 (US)
(72) Inventor: NAREN, Anjaparavanda, Memphis, Tennessee 38103 (US); LI, Chunying, Memphis, Tennessee 38120 (US); DENG, Wenlin, Cordova, Tennessee 38018 (US); JOHNSON, Leonard, Memphis, Tennessee 38127 (US); DURGAM, Gangadhar, Memphis, Tennessee 38103 (US); MILLER, Duane, Germantown, Tennessee 38139-6437 (US); TIGYI, Gabor, Memphis, Tennessee 38111 (US)
(74) Representative: de Bresser, Sara Jean
(86) International application number: PCT/US2006/027765
(87) International publication number: WO 2007/011905

(56) References cited:
- WO-A2-99/47101
- WO-A2-2005/032494
- US-A- 4 663 166
- US-A1- 2003 130 237
- US-A1- 2003 130 237
- US-A1- 2004 235 800

## Description

### Field of the Invention

The invention relates to compositions comprising one or more LPA₂ receptor agonists, as defined in the present claim 1, and their use in a method of treating or preventing diarrhea. More particularly, the invention relates to compositions of claim 1 for use in treating cystic fibrosis transmembrane conductance regulator (CFTR)-mediated diarrhea and for inhibiting activation of CFTR.

### Background of the Invention

Secretory diarrhea is often caused by infectious organisms such as *Vibrio cholerae, Clostridium difficile,* and enterotoxic *Escherichia coli.* Infectious diarrheal disease is a major cause of morbidity and mortality worldwide, and is of particular concern for children, especially in developing countries.

Cholera, for example, is an acute diarrheal illness caused by the bacterium *Vibrio cholerae.* The infection can be asymptomatic, or may produce symptoms that range from mild to severe. About five percent of infected individuals have severe disease. A hallmark of cholera is a watery diarrhea that can lead to rapid loss of body fluids, dehydration and shock. Without treatment, an affected individual may be dead within hours. Patients are most often treated through rehydration, and individuals are often administered a prepackaged combination of sugar and electrolytes admixed with water. In some areas, however, sufficient quantities of potable water may not be available for treatment. Furthermore, in severe cases of secretory diarrhea rehydration alone may not be sufficient to save the patient's life.

Cholera-associated diarrhea, for example, is induced primarily by the interaction of cholera toxin with the cystic fibrosis transmembrane conductance regulator (CFTR). CFTR is a plasma-membrane cyclic AMP-activated Cl⁻ channel expressed in a variety of tissues. In epithelial cells, CFTR mediates the secretion of chloride ions. In the intestine, CFTR-mediated Cl⁻ secretion causes the diarrhea associated with *Vibrio cholerae, Clostridium difficile,* and *Escherichia coli* infections, for example.
WO 2005/032494 discloses LPA/LPA₂ receptor agonists as well as pharmaceutical compositions which include these compounds: phosphoric acid monodecyl ester, phosphoric acid monododecyl ester, phosphoric acid monodec-9-enyl ester, phosphoric acid monododec-9-enyl ester, phosphoric acid monotetradec-9-enyl ester, phosphoric acid monotetradec-11-enyl ester, thiophosphoric acid O-decyl ester, thiophosphoric acid O-dodecyl ester, thiophosphoric acid O-tetradecyl ester, thiophosphoric acid O-dodec-9- ester, thiophosphoric acid O-tetradec-9-enyl ester, thiophosphoric acid O-octadec-9-enyl ester for the treatment of gastrointestinal perturbations that include diarrhea.
US 2003/130237 discloses a composition comprising LPA/LPA₂ agonist phosphoric acid monododecyl ester for the treatment of gastrointestinal perturbations that include diarrhea.
WO 99/47101 discloses pharmaceutical compositions comprising lysophosphatidic acid (LPA) for the treatment of gastrointestinal perturbations that include diarrhea.

Inhibition of CFTR is an attractive target for prevention and treatment of secretory diarrhea. What are needed are safe and effective compositions that can quickly stop the loss of fluids through the intestine as a result of CFTR activation.

### Summary of the Invention

The present invention relates to a therapeutically effective amount of an LPA₂ receptor agonist, a food or supplement comprising a therapeutically effective amount of an LPA₂ receptor agonist, or combinations thereof, optionally further comprising a food or supplement comprising a therapeutically effective amount of LPA for use in a method of treating or preventing diarrhea in an individual.

The invention also relates to a composition for treating diarrhea comprising an aqueous carrier comprising one or more electrolytes for rehydration and for restoring or maintaining electrolyte balance, the aqueous carrier and electrolytes being admixed with one or more LPA₂ receptor agonists.

The disclosure further relates to a therapeutically effective amount of an LPA₂ receptor agonist for use in a method of inhibiting CFTR activity in an individual.

### Brief Description of the Drawings

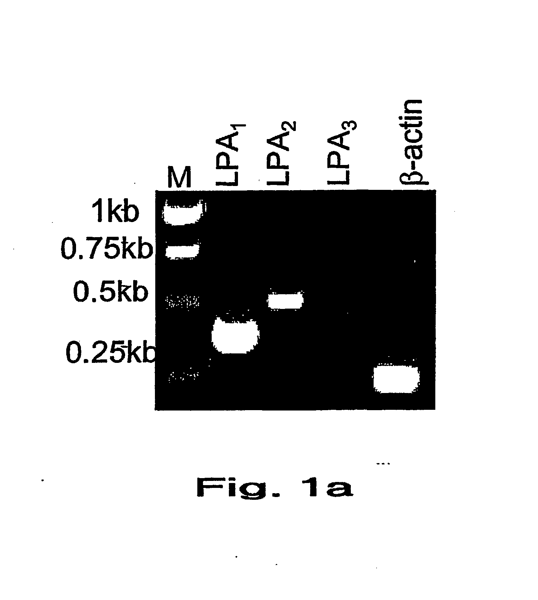

Figs. 1A through Fig. 1J illustrate that LPA₂ and NHERF2 are expressed in intestinal epithelial cells and localized primarily at or near the apical cell surfaces. (**A**) LPA₁ and LPA₂ transcripts were detected in mouse jejunum, whereas LPA₃ transcript was not detected. (β-actin was used as positive control. (**B**) LPA₁ and LPA₂ transcripts were detected in mouse ileum. RT, reverse transcriptase. (C) HT29-CL19A and Calu-3 cells express LPA₂ transcripts. (**D**) LPA₂ expression in crude plasma membrane prepared from HT29-CL19A and Calu-3 cells. (**E**) Apical localization of LPA₂ in polarized HT29-CL19A cells. CFTR immunolocalization was used as positive control and pre-immune as negative control. AP=apical, BL=basalateral. (F) LPA₂ is expressed in apical membranes of HT29-CL19A cells where CFTR also resides. (**G**) NHERF2 expression and apical localization in intestinal epithelial cells. The nucleus was stained with propidium iodide (bottom). (**H**) The PDZ motif structure in LPA receptors and their interaction with NHERF. (**I**) GST-NHEFR2 binds directly to maltose-binding protein (MBP)-C-LPA₂. (**J**) Mechanism of LPA action on LPA receptors, AC, adenylate cyclase.

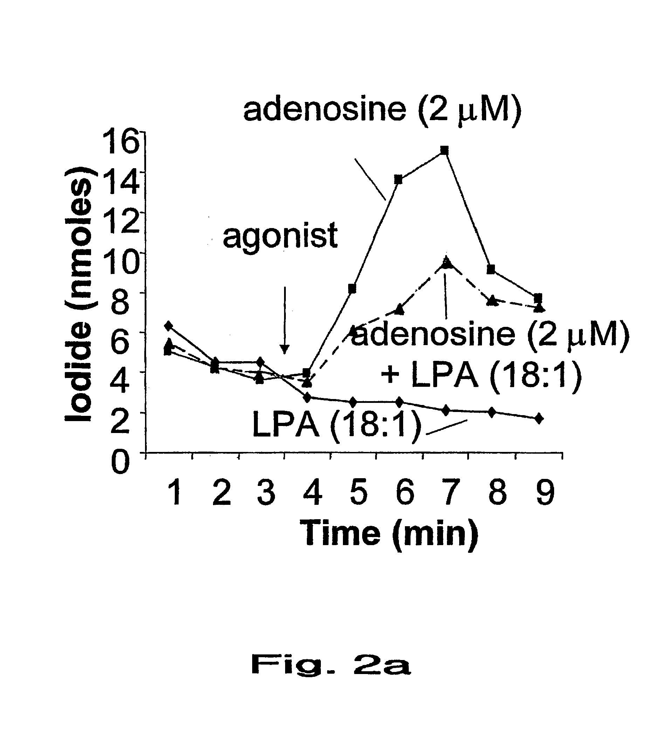

Figs. 2A through 2H illustrate that LPA inhibits CFTR-dependent Cl⁻ currents through LPA₂ receptor-mediated mechanism. (**A**) Representative cpt-cAMP activated iodide efflux in Calu-3 cells treated with LPA 18:1 (20 µM). (**B**) Iodide efflux in Calu-3 cells pretreated with pertussis toxin (PTX). **P* < 0.05 for a Student's *t* test (mean ± SEM, n=3). (**C**) Peak efflux of iodide in LPA (18:1)-treated Calu-3 cells in response to adenosine. **P* < 0.05 (n=3). (**D**) Peak efflux of iodide in response to adenosine in Calu-3 cells treated with various LPA analogs (20 µM). **P* < 0.05; **P < 0.01 (n=3). (**E**) Representative traces of short-circuit currents (I_{sc}) in response to adenosine (ADO) in HT29-CL19A cells without (top) or with (bottom) LPA (20:4) pretreatment. (**F**) I_{sc} in response to ADO in Calu-3 cells. (**G**) I_{sc} in response to cAMP in isolated mouse intestine. **(H)** Peak CFTR-mediated currents (% control) in isolated mouse intestine in the presence of LPA (20:4). ** *P* < 0.01 (n=4).

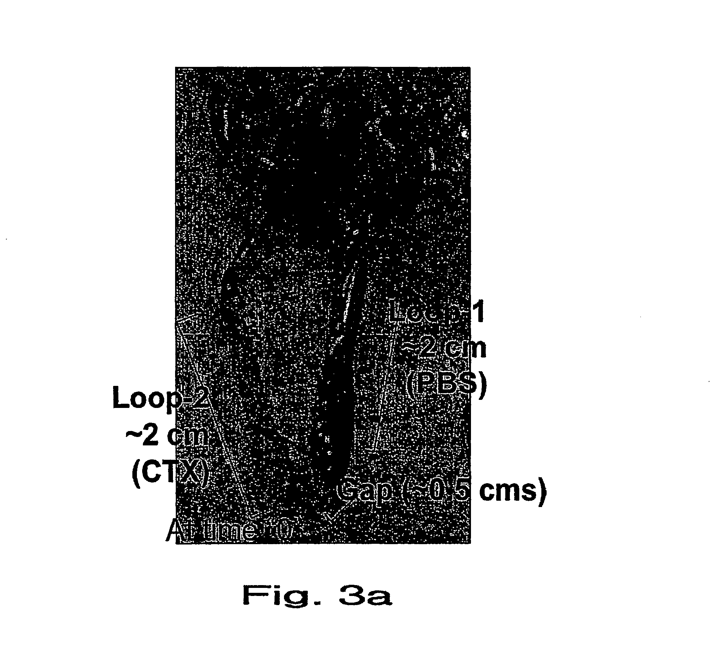

Figs. 3A through 3E demonstrate that LPA inhibits CFTR-dependent intestinal fluid secretion induced by cholera toxin. (**A**) Surgical procedure of mouse ileal loop formation. (**B**) Ileal loops excised 6 h after luminal injection. (**C**) Representative loops 6 h after luminal injection with CTX (1 to 100 µg, top). The bar graph (bottom) shows the averaged ileal loop weight. ** *P* < 0.01; *** *P* < 0.001 (n=3). (**D**) Isolated mouse ileal loops 6 h after luminal injection of 1.0 µg CTX without (top) and with (bottom) 40 µM LPA (20:4). (E) Ileal loop weight at 6 h injected with CTX and LPA (20:4). * *P* < 0.05; ** *P* < 0.01 (n=3).

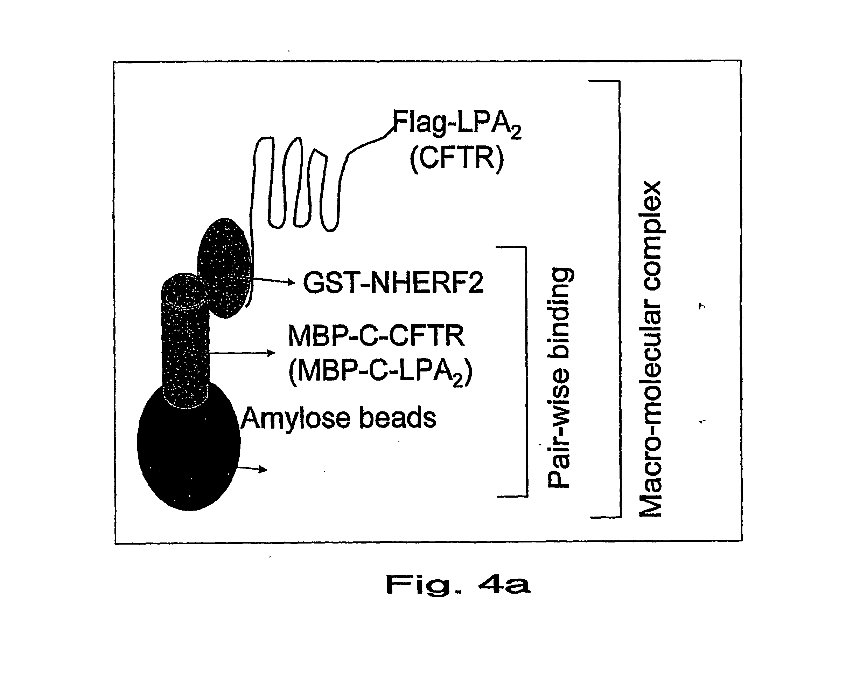

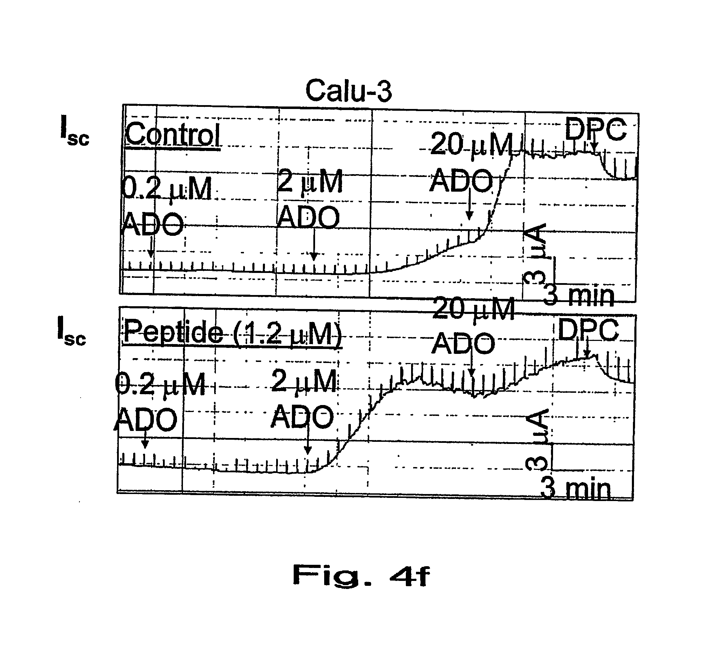

Figs. 4A through 4I demonstrate that LPA₂ forms a macromolecular complex with CFTR mediated through NHERF2 and that disrupting the complex using a LPA₂-specific peptide reverses LPA₂-mediated CFTR inhibition. (A) A pictorial representation of the macromolecular complex assay. (**B**) A macromolecular complex of MBP-C-CFTR, GST-NHERF2, and Flag-tagged LPA₂. (C) A macromolecular complex ofMBP-C-CFTR, GST-NHERF2, and Flag-tagged LPA₂, LPA₂-ΔSTL, or LPA₂-L351A. WT, wild type. (**D**) BHK or BHK-CFTR cells transiently transfected with LPA₂ were cross-linked using 1 mM DSP and co-immunoprecipitated using anti-CFTR antibody (NBD-R) and probed for LPA₂. (**E**) Calu-3 cell lysates were co-immunoprecipitated using anti-CFTR antibody (R1104) and probed for NHERF2 and LPA₂. (**F**) I_{sc} in response to ADO in Calu-3 cells without (top) or with (bottom) LPA₂-specific peptide delivered before I_{sc} measurement. Cells in both groups were pretreated with 25 µM LPA (20:4) for 30 min before activating with ADO. (**G**) Immunofluorescence micrographs of Calu-3 cells from Fig. 4F showing the LPA₂-specific peptide delivered (bottom). Note: Calu-3 cells express endogenous LPA₂ (top). (**H**) NHERF2 binding to LPA₂ in the presence of LPA₂-specific peptide. **P* < 0.05; ** *P* < 0.01 compared with control (n=3). (**I**) A schematic view of LPA inhibition on CFTR-dependent Cl⁻ transport.

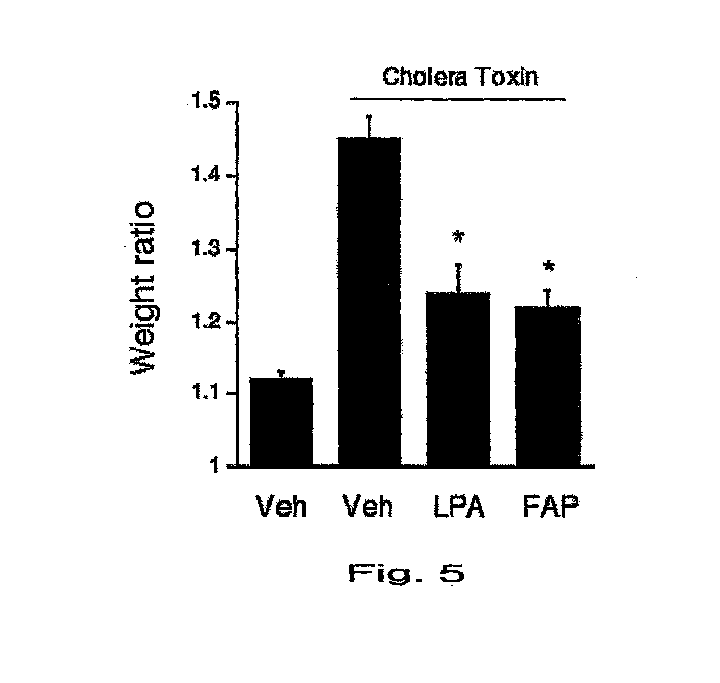

Fig. 5 is a graph illustrating the effects of CTX-induced secretion, as measured by the weight ratio of the small intestine before and after flushing of its content. Measurements were made and ratios calculated for four groups: buffer alone, buffer with cholera toxin (CTX), buffer with CTX and LPA, and buffer with CTX and FAP 18:1d9. * *P* < 0.05.

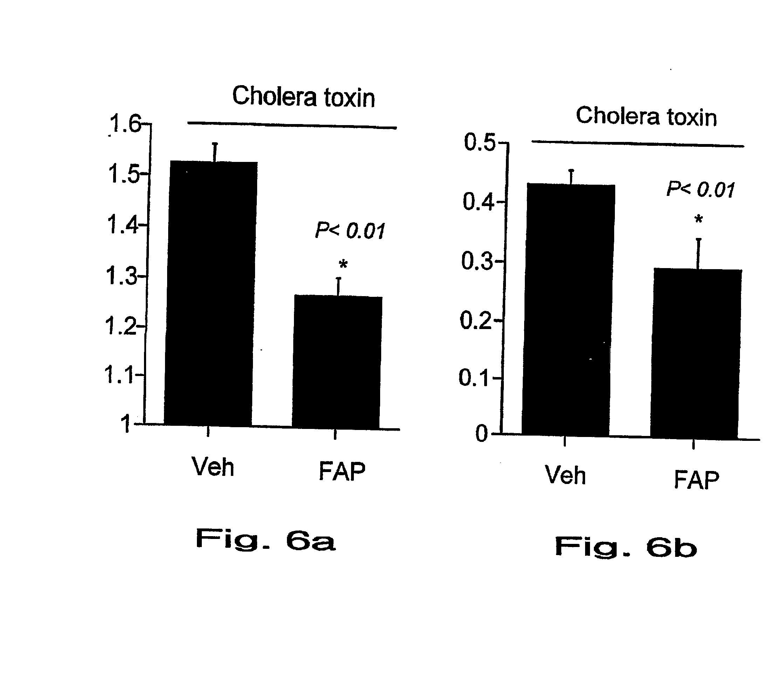

Fig. 6 is a graph illustrating the intestinal weight ratio and the volume of intestinal secretion for buffer with cholera toxin (CTX) and buffer with CTX and FAP 18:1d9. * *P* < 0.05.

### Detailed Description

The inventors have discovered that lysophosphatidic acid (LPA) inhibits cholera toxin-induced secretory diarrhea through CFTR-dependent protein interactions. Furthermore, the inventors have discovered that the mechanism by which LPA inhibits CFTR involves the LPA₂ receptor and that LPA₂ receptor agonists synthesized by the inventors provide compositions for preventing and treating CFTR-mediated secretory diarrhea. The inventors have also discovered that LPA and LPA₂ receptor agonists can inhibit CFTR-dependent Cl⁻ currents in response to adenosine activation. Bacterial diarrhea is most often the result of interaction of bacterial toxins with CFTR, but diarrhea can also result from an increased immunoinflammatory response triggered by cytokines or mediators such as adenosine secreted from the intestinal mucosal inflammatory cells in response to luminal factors. The invention therefore provides compositions for treating secretory diarrhea of bacterial origin (e.g. bacterial infection caused by one or more of *Vibrio cholerae, Clostridium difficile,* and *Escherichia coli*)*,* as well as diarrhea induced by stress and/or an immunoinflammatory response.

Compositions as described by the invention may be utilized for therapy in animals, particularly mammals, such as humans, non-human primates, horses, cows, pigs, goats, sheep, dogs, and cats, for example. Compositions described herein are described as compositions comprising LPA₂ receptor agonists, but it should be understood by those of skill in the art that such compositions may further comprise LPA, a food or supplement comprising LPA, one or more LPA₂ receptor agonists, or combinations thereof. LPA may be found, for example, in dietary supplements.

Treatment for diarrheal illness may be administered prior to onset of symptoms (e.g., following exposure to one or more organisms known to cause secretory diarrhea) or after onset of symptoms. Compositions of the invention may be distributed to groups of individuals in areas where bacterial or viral epidemics, for example, are occurring, to prevent fluid loss and associated electrolyte imbalance.

Compounds have been identified by the inventors which are specific for certain LPA receptors, may bind to more than one LPA receptor in a relatively non-specific manner, or may bind to more than one LPA receptor but preferentially bind to certain of the LPA receptors. Compounds of the present invention will therefore comprise compounds that bind to the LPA₂ receptor, but even more preferably will comprise compounds that preferentially bind to the LPA₂ receptor. Compounds of the invention comprise those that act as agonists of the LPA₂ receptor. Therapeutically effective amounts are amounts that produce sufficient inhibition of CFTR to decrease symptoms (e.g., fluid accumulation in the intestine and fluid loss through diarrhea) associated with CFTR activation.
Viewed from a first aspect, the present invention therefore provides a composition comprising one or more LPA₂ receptor agonists for use in a method of treating or preventing diarrhea wherein the one or more LPA₂ receptor agonists are selected from the group consisting of (R)-octanoic acid 1-octanoyloxymethyl-2-thiophosphonooxy-ethyl ester, (*S*)-octanoic acid 1-octanoyloxymethyl-2-thiophosphonooxy-ethyl ester, (*R*)-thiophosphoric acid O-(2,3-bis-octyloxy-propyl) ester, mono potassium thiophosphoric acid O-(2-heptadec-9-enyl-(1,3)-dioxolan-4-ylmethyl) ester, mono potassium phosphoric acid O-(2-heptadec-9,12,15-trienyl-(1,3)-dioxolan-4-ylmethyl) ester, (2R,4S) mono potassium thiophosphoric acid O-(2-heptadec-9-enyl-(1,3)-dioxolan-4-ylmethyl) ester, (2S,4S) mono potassium thiophosphoric acid O-(2-heptadec-9-enyl-(1,3)-dioxolan-4-ylmethyl) ester, (2R,4R) mono potassium thiophosphoric acid O-(2-heptadec-9-enyl-(1,3)-dioxolan-4-ylmethyl) ester, (2R,4R) mono potassium phosphoric acid O-(2-heptadec-9-enyl-(1,3)-dioxolan-4-ylmethyl) ester, (2S,4R) mono potassium thiophosphoric acid O-(2-heptadec-9-enyl-(1,3)-dioxolan-4-ylmethyl) ester, and combinations thereof.
Viewed from a further aspect, the present invention provides a composition comprising:
an aqueous carrier,
one or more LPA₂ receptor agonists; and
one or more electrolytes.
wherein the one or more LPA₂ receptor agonists are selected from (R)-octanoic acid 1-octanoyloxymethyl-2-thiophosphonooxy-ethyl ester, (S)-octanoic acid 1-octanoyloxymethyl-2-thiophosphonooxy-ethyl ester, (R)-thiophosphoric acid O-(2,3-bis-octyloxy-propyl) ester, mono potassium thiophosphoric acid O-(2-heptadec-9-enyl-(1,3)-dioxolan-4-ylmethyl) ester, mono potassium phosphoric acid O-(2-heptadec-9,12,15-trienyl-(1,3)-dioxolan-4-ylmethyl) ester, (2R,4S) mono potassium thiophosphoric acid O-(2-heptadec-9-enyl-(1,3)- dioxolan-4-ylmethyl) ester, (2S,4S) mono potassium thiophosphoric acid O-(2- heptadec-9-enyl-(1,3)-dioxolan-4-ylmethyl) ester, (2R,4R) mono potassium thiophosphoric acid O-(2-heptadec-9-enyl-(1,3)-dioxolan-4-ylmethyl) ester, (2R,4R) mono potassium phosphoric acid O-(2-heptadec-9-enyl-(1,3)-dioxolan-4-ylmethyl) ester, (2S,4R) mono potassium thiophosphoric acid O-(2-heptadec-9-enyl-(1,3)- dioxolan-4-ylmethyl) ester, and combinations thereof.

Compounds listed in Table 1 and Table 2 have been synthesized by the inventors and are example of compounds that may be used in compositions for use in the treatment described herein. The first 12 compounds of Table 1 may each optionally further be present in the composition of the invention. Methods of synthesis of these compounds are described in United States Patent Number 6,875,757 (Miller et al.*)* and United States Patent Application Number 10/963,085 (Publication Number 20060009507; Miller et al*).*

**Table 1**

| **STRUCTURE** | **CHEMICAL NAME** | **LPA₂ EC₅₀ (Eₘₐₓ) nM** |
|---|---|---|
| 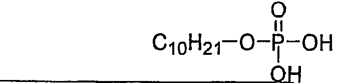 | Phosphoric acid monodecyl ester | 1800 (82) |
| 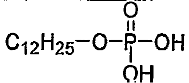 | Phosphoric acid monododecyl ester | 3100 (50) |
| 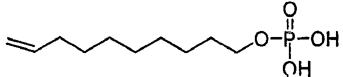 | Phosphoric acid monodec-9-enyl ester | 3800 (100) |
| 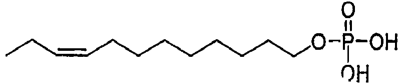 | Phosphoric acid monododec-9-enyl ester | 717(78) |
| 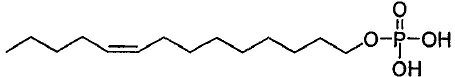 | Phosphoric acid monotetradec-9-enyl ester | 397 (58) |
| 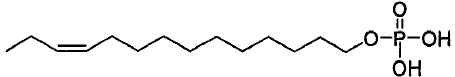 | Phosphoric acid monotetradec-11-enyl ester | 4100 (75) |
| 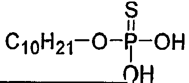 | Thiophosphoric acid O-decyl ester | 4570(100) |
| 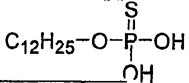 | Thiophosphoric acid O-dodecyl ester | 1000 (100) |
| 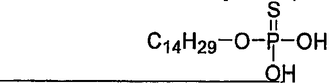 | Thiophosphoric acid O-tetradecyl ester | 2500 (100) |
| 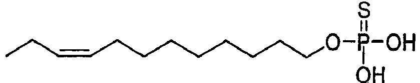 | Thiophosphoric acid O-dodec-9-enyl ester | 677 (100) |
| 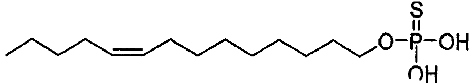 | Thiophosphoric acid O-tetradec-9-enyl ester | 480 (150) |
| 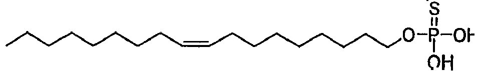 | Thiophosphoric acid O-octadec-9-enyl ester | 244 (175) |
| 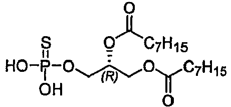 | (R)-Octanoic acid 1-octanoyloxymethyl-2-thiophosphonooxy-ethyl ester | 6330 (58) |
| 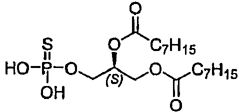 | (*S*)-Octanoic acid 1-octanoyloxymethyl-2-thiophosphonooxy-ethyl ester | 7170(17) |
| 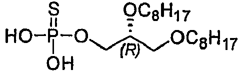 | (*R*)-Thiophosphoric acid O-(2,3-bis-octyloxy-propyl) ester | 5720 (27) |

**Table 2**

| **STRUCTURE** | **CHEMICAL NAME** | **LPA₂ EC₅₀ (E_{MAX})nM** |
|---|---|---|
| 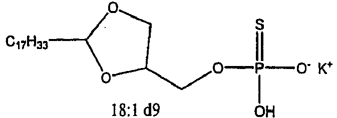 | Mono potassium thiophosphoric acid O-(2-heptadec-9-enyl-(1,3)-dioxolan-4-ylmethyl) ester | 1170(87) |
| 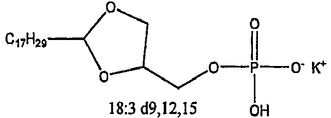 | Mono potassium phosphoric acid O-(2-heptadec-9,12,15-trienyl-(1,3)-dioxolan-4-ylmethyl) ester | 1710 (51) |
| 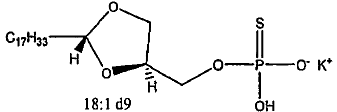 | (2R,4S) Mono potassium thiophosphoric acid O-(2-heptadec-9-enyl-(1,3)-dioxolan-4-ylmethyl) ester | 1540 (72) |
| 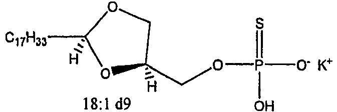 | (2S,4S) Mono potassium thiophosphoric acid O-(2-heptadec-9-enyl-(1,3)-dioxolan-4-ylmethyl) ester | 1320 (87) |
| 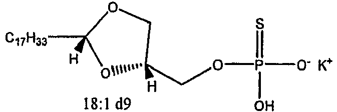 | (2R,4R) Mono potassium thiophosphoric acid O-(2-heptadec-9-enyl-(1,3)-dioxolan-4-ylmethyl) ester | 1090 (85) |
| 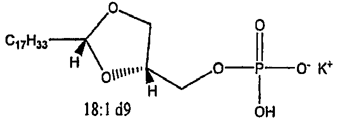 | (2R,4R) Mono potassium phosphoric acid O-(2-heptadec-9-enyl-(1,3)-dioxolan-4-ylmethyl) ester | 1710 (42) |
| 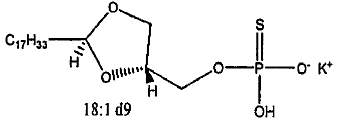 | (2S,4R) Mono potassium thiophosphoric acid O-(2-heptadec-9-enyl-(1,3)-dioxolan-4-ylmethyl) ester | 1300 (77) |

Of these compounds in Tables 1 and 2, thiophosphoric acid O-dodecyl ester, thiophosphoric acid O-tetradecyl ester, thiophosphoric acid O-dodec-9-enyl ester, thiophosphoric acid O-tetradec-9-enyl ester, and thiophosphoric acid O-octadec-9-enyl ester have been demonstrated to be particularly effective for binding to the LPA₂ receptor and thereby inhibiting CFTR activation and thus may preferably be further present in the compositions of the invention.

Therapeutic formulations can be administered by a variety of routes including, for example, oral, rectal, transdermal, subcutaneous, intravenous, intramuscular, and intranasal, as well as administration via nasogastric tube.

Compositions of the present invention comprise at least one LPA₂ receptor agonist preferably provided in conjunction with an acceptable pharmaceutical carrier, e.g. an aqueous carrier. In one embodiment, for example, such a composition may comprise at least one LPA₂ receptor agonist admixed in an aqueous carrier comprising sugar and electrolytes suitable for rehydration and maintaining or restoring electrolyte balance in an individual who has experienced an episode of secretory diarrhea. In another embodiment, one or more LPA₂ receptor agonists may be provided in a powder comprising one or more electrolytes, and, optionally, sugar. Compositions may also comprise antibiotics, anti-inflammatory agents, or other agents that may act to provide a benefit to an individual at risk for or experiencing secretory diarrhea. Compositions of the invention are described for oral administration, and it is well within the skill of those in the art to prepare oral rehydration salt (ORS) comprising one or more LPA₂ receptor agonists admixed with, or provided in conjunction with, the ORS. Pharmaceutically acceptable compositions for IV administration may be required for treatment of more serious cases of secretory diarrhea. Such compositions may comprise, for example, saline solutions containing therapeutically effective amounts of LPA₂ receptor agonists or Ringer's lactate solutions containing therapeutically effective amounts of LPA₂ receptor agonists. The formulation may be in dry or liquid form, or as an oral or intravenous sugar-electrolyte solution or dry composition.

For oral administration, Rehydron™ (Orion Pharma International, Finland) or Pedialyte™ (Ross, USA) solution can be administrated according to the manufacturer's instructions, and may be admixed with one or more of the LPA₂ receptor agonists according to the invention prior to administration. In yet another embodiment, a parenteral rehydration solution may contain, for example, (in addition to one or more LPA₂ receptor agonists according to the invention), glucose, sodium chloride and potassium chloride as the one or more electrolytes. Administration of an electrolyte for rehydration therapy may be performed as recommended by the World Health Organization.. (See, for example, World Health Organization, Diarrheal Diseases Control Program: A Manual For The Treatment Of Acute Diarrhea For Use By Physicians And Other Senior Health Workers. Geneva: WHO, 1984:WHO/CDD/SER/80.2(rev.1); World Health Organization; The Treatment Of Diarrhea: A Manual For Physicians And Other Senior Health Workers, available online at http://www.who.int.) Oral rehydration therapy may additionally comprise alanine, arginine, vitamin A, and zinc, for example, to increase the rate of repair of the intestinal epithelia.

Compositions of the invention may also contain additional ingredients such as buffering agents, preservatives, compatible carriers, and optionally additional therapeutic agents such as antibiotics. Suitable buffering agents include: acetic acid, citric acid, boric acid, phosphoric acid and salts thereof Compositions may additionally comprise pharmaceutically acceptable preservatives.

In making the formulations of the present invention, the LPA₂ receptor agonist is usually admixed with an excipient, diluted in an excipient or enclosed within a carrier that can be in the form of a capsule, sachet, or paper, for example. When the excipient serves as a diluent, it can be a solid, semi-solid, or liquid material, which acts as a vehicle, carrier or medium for the active ingredient. Compositions provided herein may be in the form of tablets, pills, powders, lozenges, sachets, cachets, elixirs, suspensions, emulsions, solutions, syrups, aerosols (as a solid or in a liquid medium), ointments containing for example up to 10% by weight of the one or more LPA₂ receptor agonists, soft and hard gelatin capsules, suppositories, sterile injectable solutions, and sterile packaged powders. Compositions can be administered in the form quiescently frozen confections, ice milks, ice creams, frozen yogurts, or other frozen treats, for example. Compositions may also be administered as flavored drinks such as fruit-flavored drinks, juices, or drinks containing at least a percentage of fruit juice.

LPA₂ receptor agonists may be milled to a suitable particle size prior to combining or admixing with other ingredients. If the LPA₂ receptor agonist is substantially insoluble, it may be is milled to a particle size of less than 200 mesh. If the LPA₂ receptor agonist is substantially water soluble, the particle size may be adjusted by milling to provide a substantially uniform distribution in the formulation, for example about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. Compositions of the present invention can be formulated so as to provide relatively immediate, sustained, or delayed release of the active ingredient after administration to the patient by employing procedures known to those of skill in the art.

Compositions of the invention may be provided in a unit dosage form or provided in bulk quantity comprising sufficient volume for multiple doses, each dosage containing from about 2.0 mg to about 1000 mg, more usually about 20 mg to about 500 mg, of at least one LPA₂ receptor agonist. The term "unit dosage form" refers to physically discrete units suitable as unitary dosages for human subjects and other mammals, each unit containing a predetermined quantity of active material calculated to produce the desired therapeutic effect, in association with a suitable pharmaceutical excipient.

For preparing solid compositions such as tablets the one or more LPA₂ receptor agonists are mixed with a pharmaceutical excipient to form a solid formulation preferably containing a homogeneous mixture of the one or more LPA₂ receptor agonists to form a composition of the present invention. When referring to the formulation as homogeneous, it is meant that the LPA₂ receptor agonist is dispersed relatively evenly throughout the composition so that the composition may be readily subdivided into equally effective unit dosage forms. In some embodiments, for example, tablets or caplets may be provided, the tablets or caplets being scored to facilitate use of one-fourth, one-half, etc. of a tablet or caplet (for use in children, for example).

Tablets or pills of the present invention may be coated or otherwise compounded to provide a dosage form providing the advantage of modified or extended release. For example, the tablet or pill can comprise an inner dosage and an outer dosage component, the latter being in the form of an envelope over the former. The two components can be separated by enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

LPA₂ receptor agonists may also be provided in the form of a transdermal delivery device. Such transdermal "patches" may be used to provide continuous or discontinuous infusion of the LPA₂ receptor agonist in controlled amounts. The construction and use of transdermal patches for the delivery of pharmaceutical agents is well known in the art and is described in, for example, U.S. Pat. No. 5,023,252. Such patches may be constructed for continuous, pulsatile, or on demand delivery of pharmaceutical agents.

Injectable drug formulations include, for example, solutions, suspensions, gels, microspheres and polymeric injectables, and can comprise excipients such as solubility-altering agents (for example, ethanol, propylene glycol and sucrose) and polymers (for example, polycaprylactones and PLGA's).

Compositions suitable for parenteral administration may comprise a sterile aqueous preparation of the LPA₂ receptor agonist, which is preferably isotonic with the blood of the recipient. This aqueous preparation may be formulated according to known methods using those suitable dispersing or wetting agents and suspending agents. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono or di-glycerides. In addition, fatty acids such as oleic acid may be used in the preparation of injectables. Carrier formulations suitable for oral, subcutaneous, intravenous, intramuscular, etc. can be found in Remington's Pharmaceutical Sciences, Mack Publishing Company, Easton, Pa.

Timed-release, delayed release or sustained release delivery systems may increase ease of administration and efficacy of compositions as provided by the invention. Such delivery systems are commercially available and known to those of ordinary skill in the art. They include polymer based systems such as polylactic and polyglycolic acid, polyanhydrides and polycaprolactone; nonpolymer systems that are lipids including sterols such as cholesterol, cholesterol esters and fatty acids or neutral fats such as mono-, di- and triglycerides; hydrogel release systems; silastic systems; peptide based systems; wax coatings, compressed tablets using conventional binders and excipients, partially fused implants. Specific examples include: (a) erosional systems in which the polysaccharide is contained in a form within a matrix, found in U.S. Pat. No. 4,452,775 to Kent; U.S. Pat. No. 4,667,014 to Nestor et al; and U.S. Pat. No. 4,748,034 and U.S. Pat. No. 5,239,660 to Leonard; and (b) diffusional systems in which an active component permeates at a controlled rate through a polymer, found in U.S. Pat. No. 3,832,253 to Higuchi et al. and U.S. Pat. No. 3,854,480 to Zaffaroni. In addition, a pump-based hardware delivery system can be used, some of which are adapted for implantation.

Use of a long-term sustained release implant may be particularly suitable for treatment of diarrhea in immunodeficient patients, who may need continuous administration of the compositions of the present invention. "Long-term" release, as used herein, means that the therapeutic agent is delivered at therapeutic levels of the agent for at least 30 days, and preferably 60 days. Long-term sustained release compositions and devices are well known to those of ordinary skill in the art.

Administration of the LPA₂ receptor agonist, or formulations containing one or more LPA₂ receptor agonists, can be provided as either a single dosage treatment, if suitable, or a plurality of dosages that are repeated, e.g., at regular intervals. Periodic administration can be two or more times per day, on two or more consecutive or non-consecutive days, etc.

The present disclosure also provides the compositions of the invention for use in a method of inhibiting activity of a cAMP activated chloride channel (CFTR). This involves providing to an individual a therapeutically effective amount of at least one LPA₂ receptor agonist composition according to the invention, the amount of the LPA₂ receptor agonist being effective to inhibit formation of a macromolecular complex between the LPA₂ receptor, the CFTR, and a Na⁺/H⁺ exchanger regulatory factor-2 (NHERF-2), thereby inhibiting activity of the CFTR.

The compositions of the disclosure may be used in research reagents and kits for studying CFTR-mediated cellular functions and pathways. Kits may comprise, for example, one or more aliquots of a composition according to the invention, optionally in combination with, cell culture media, buffers, or other reagents to facilitate culture of cells with at least one LPA₂ receptor agonist or introduction of one or more LPA₂ receptor agonists into the cellular environment.

### EXAMPLES

The invention may be further described by means of the following examples.

### Tissue culture and transfection

HT29-CL19A (human colonic epithelial origin), Calu-3 (serous gland epithelial cells), and BHK (baby hamster kidney cells) were cultured as described (26). For Ussing chamber experiments, HT29-CL19A and Calu-3 cells were grown on permeable filters. EVOM Epithelial Voltohmmeter (World Precision Instruments; Sarasota, FL) was used to measure the transepithelial resistances on a daily basis. BHK cells were transiently transfected with Flag-LPA receptors using a vaccinia virus expression system (*27*).

### Antibodies, reagents, and constructs

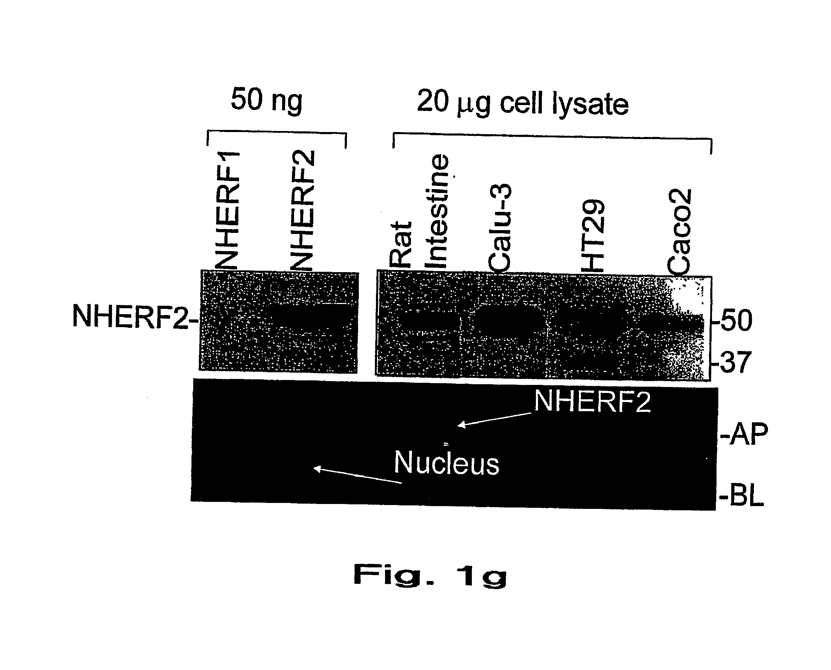

CFTR antibodies R.1104 monoclonal mouse antibody and NBD-R polyclonal rabbit antibody have been described previously (28). We generated peptide-specific anti-LPA₂ antibody (rabbit-2143) against the last 11 amino acids (a.a. 341-351) of LPA₂ (Genemed Synthesis, CA) which was affinity-purified using Protein-A column. Anti-NHERF2 antibody was a kind gift from Dr. Emanuel Strehler (Mayo Clinic, Rochester, MN). We also generated our own NHERF2-specific antibody (rabbit-2346) against the full-length NHERF2 protein (Genemed Synthesis, CA), which was affinity purified using Protein-A column and the NHERF2 antigen itself. This isoform-specific antibody for NHERF2 does not cross-react with NHERF1, which shares approximately 50% sequence identity with NHERF2 (Fig. 1G, upper left panel of the main paper). Anti-Flag mab was obtained from Sigma Chem. Co. (St. Louis, MO). Maltose binding protein (MBP)-fusion proteins for C-terminal tails of LPA receptors, i.e., LPA₁ (a.a. 316 to 364), LPA₂ (a.a. 298 to 351), and LPA₃ (a.a. 298 to 353), were generated using pMAL vectors (New England Biolabs; Beverly, MA). Flag-tagged LPA₂, LPA₂-ΔSTL, and LPA₂-L351A were generated using pCDNA-3 vector (Invitrogen; Carlsbad, California) and QuickChange mutagenesis kit (Strategene; La Jolla, CA). The peptide delivery system (Chariot™) was procured from Active Motif (Carlsbad, CA). LPA (18:1), S1P, and PA were purchased from Avanti Polar Lipids, Inc. (Alabaster, AL). Other lipids, LPA (20:4) and LPA (18:2), have been described (*29*). Cholera toxin and pertussis toxin were obtained from Sigma. Other reagents have been described previously (*26*).

### Iodide efflux measurements

Iodide efflux was performed as described (*30*). Calu-3 and HT29-CL19A cells were grown to confluency in 60-mm dishes and then pretreated with or without LPA 18:1, 18:2, 20:4 (complexed with BSA), or a structurally related lysophospholipid, sphingosine-1-phosphate (S1P), for 20 min before iodide efflux measurement. The first four aliquots were used to establish a stable base line in efflux buffer alone. Agonists, cpt-cAMP (200 µM) or adenosine (2 µM), were added to the efflux buffer with or without various LPA, and samples were collected every minute for 6 min in the continued presence of agonists and LPA (i.e., the efflux buffer used for subsequent replacements also contained agonists and LPA at the same concentration). The iodide concentration of each aliquot was determined using an iodide-sensitive electrode (Orion Research Inc., Boston, MA) and converted to iodide content (i.e., the amount of iodide released during the 1-min interval in nanomoles). For some studies, the peak efflux of iodide (5 min time point) was presented as the percentage of control. For the pertussis toxin (PTX) experiments, the cells were pretreated with 100 ng/ml, or 200 ng/ml of PTX for 24 h at 37°C incubator before iodide efflux measurement.

### Short-circuit current (I_{sc}) measurements

Calu-3 and HT29-CL19A polarized cell monolayers were grown to confluency on Costar Transwell permeable supports (filter area is 0.33 cm²). Filters were mounted in an Ussing chamber, and short-circuit currents (I_{sc}) mediated through CFTR Cl channel were carried out as described previously (*31*). Epithelia were bathed in Ringer's solution (mM) *(serosal*/*basolateral:* 140 NaCl, 5 KCl, 0.36 K2HPO₄, 0.44 KH₂PO₄, 1.3 CaCl₂, 0.5 MgCl₂, 4.2 NaHCO₃, 10 Hepes, 10 glucose, pH 7.2, [Cl⁻] = 149), and low Cl-Ringer's solution (mM) *(luminal*/*apical:* 133.3 Nagluconate, 5 K-gluconate, 2.5 NaCl, 0.36 K2.HPO₄, 0.44 KH₂PO₄, 5.7 CaCl₂, 0.5 MgCl₂, 4.2 NaHCO₃, 10 Hepes, 10 mannitol, pH 7.2, [Cl⁻] = 14.8) at 37 °C, gassed with 95% O₂ and 5%) was stripped of serosal and smooth muscle layers (32) before being mounted in a Ussing chamber. LPA (20:4; 0-35 µM) was added into both the luminal (apical) side and serosal (basolateral) side of the isolated mouse intestine for 30 min. Increasing concentrations of cpt-cAMP (0-100 µM) or ADO (0-100 µM) were applied to both sides to elicit a CFTR-dependent Cl current response. DPC (500 µM, added into luminal side) was used to inhibit the Cl currents towards the end of the experiment. CO₂. LPA (20:4) was added into both the luminal (apical) side and serosal (basolateral) side of the cell monolayers for 30 min before adenosine (ADO) was added into the luminal/apical side. In parallel, some filters were also pretreated for 30 min with phosphatidic acid (PA; 0-35 µM) and sphingosine-1-phosphate (S1P; 0-35 µM) before ADO addition. CFTR Cl⁻ channel inhibitor, diphenylamine-2-carboxylate (DPC; 500 µM; added into luminal side), was used to inhibit the Cl⁻ currents towards the end of the experiment. To demonstrate the effect of LPA on Cl⁻ transport in the mucosa, a patch of mouse intestine (0.112 cm²).

### Intestinal fluid secretion (in vivo) experiments

The method of Verkman's group was followed with modifications (33) with Institutional Animal Care and Use Committee approval. CD1 mice were held off food 24 h prior to inducing anesthesia using pentobarbital (60 mg/kg). Body temperature of the mice was maintained at 36-38 °C during surgery using a circulating water heating pad. A small abdominal incision was made to expose the small intestine. Ileal loops (~20 mm) proximal to the cecum were exteriorized and isolated (two loops per mouse: 1 loop PBS, 1 loop PBS + toxin). The loops were then injected with 100 µl of PBS alone or PBS containing cholera toxin (1-100 µg). Both the control and cholera toxin-injected loops were tested in the presence and absence of LPA (10-40 µM). The abdominal incision and skin incision were closed with wound clips, and the mice were allowed to recover. Intestinal loops were collected in a terminal procedure 6 h later. PA (10-100 µM) and S1P (10-100 µM) were also used in parallel experiments as described above.

### Macromolecular complex assembly

This assay (*34*) was performed using maltose binding protein (MBP)-CFTR-C tail fusion protein (1 µM) immobilized on amylose beads (20 µl) and incubated with GST-NHERF2. This step, which is called pairwise binding, was done in 200 µl of lysis buffer (PBS-0.2% Triton-X 100 + protease inhibitors) and mixed at 22 °C for 2 h. The complex was washed once with the same buffer and allowed to bind Flag-tagged LPA₂ from lysates of BHK cells expressing the receptor. The binding was done at 4 °C for 3 h with constant mixing. The complex was washed with lysis buffer, eluted with sample buffer, and analyzed by immunoblotting using anti-Flag monoclonal antibody.

### Delivery of LPA₂ receptor-specific peptide and immunostaining

Delivery of LPA₂-specific peptide was performed using the Chariot™ system according to the manufacturer's instructions (Active Motif; Carlsbad, CA) (*35*). Briefly, 1.2 µM peptide containing the PDZ motif of LPA₂ (last 11 amino acids; a.a. 341 to 351, ENGHPLMDSTL) was mixed with Chariot solution (total volume: 400 µl) at room temperature for 30 min, then the Chariot-peptide complex was added to both luminal and serosal sides of polarized Calu-3 and HT29-CL19A cells grown on permeable supports and incubated for 1 h at 37 °C in a humidified atmosphere containing 5% CO₂ before mounting in a Ussing chamber. At the end of the experiment, the epithelial cells were fixed and immunostained for the efficiency of the peptide delivery using anti-LPA₂ antibody (raised against the last 11 amino acids of LPA₂) that also recognizes this LPA₂-specific peptide and subjected the fixed cells to immunofluorescence confocal microscopy. Other peptide (CFTR a.a. 107-117) was also delivered as described above as negative control.

### Data analysis

Results are presented as mean ± SEM for the indicated number of experiments. The unpaired Student's *t* test was used for statistical comparison of mean values between two experimental groups. A value of *P* < 0.05, *P* < 0.01, or *P* < 0.001 was considered statistically significant.

### Example 1 - Localization of LPA₂ Receptors in Intestinal Tissue

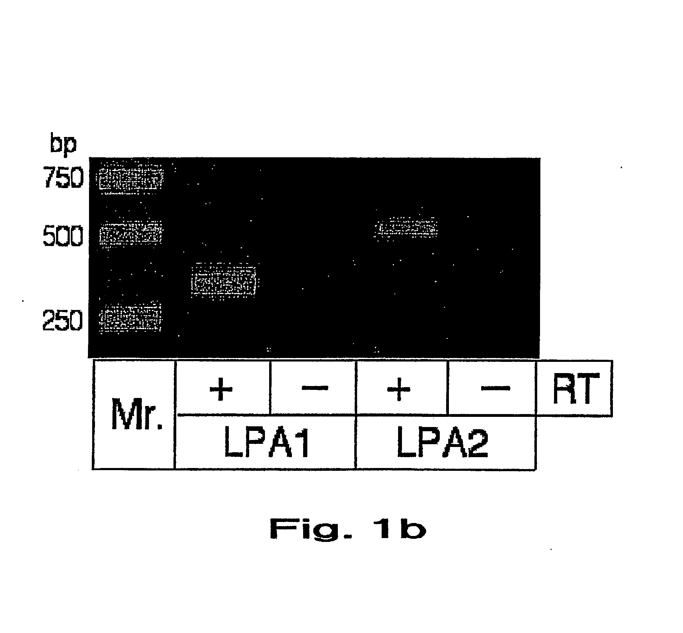

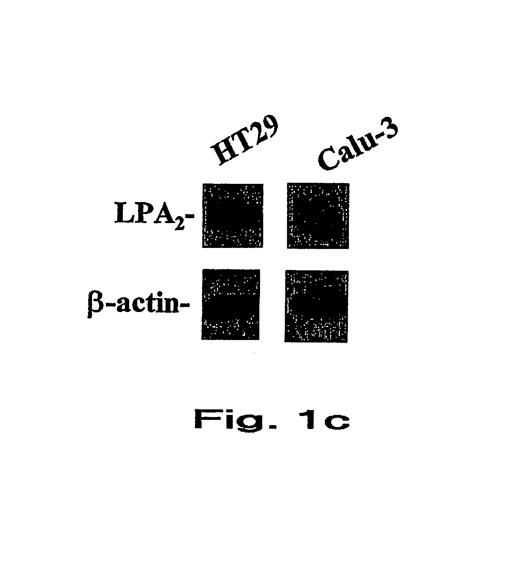

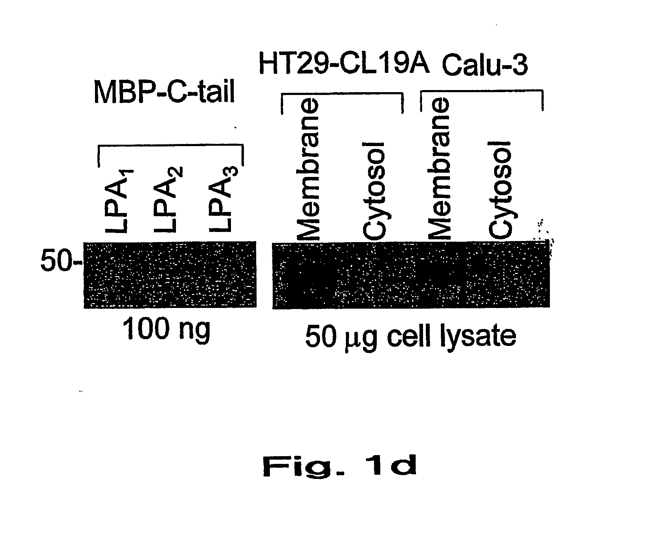

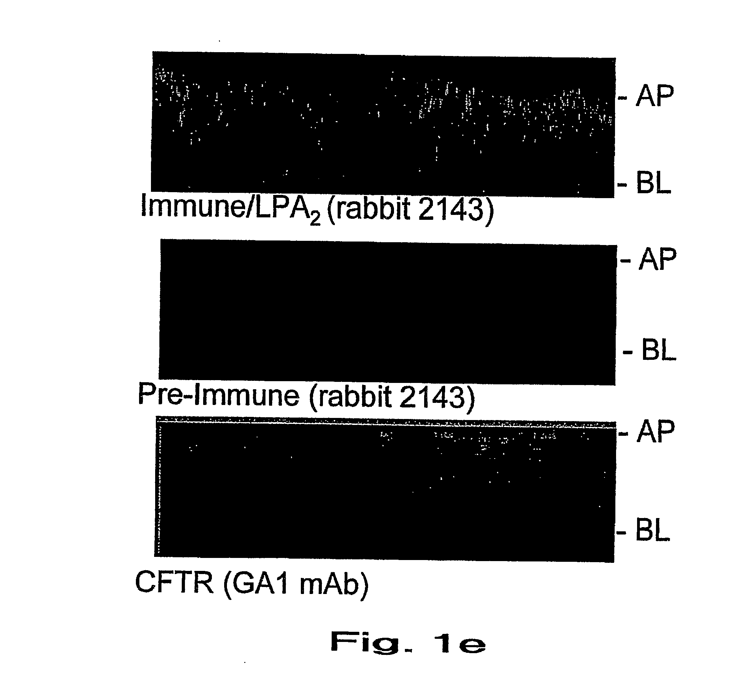

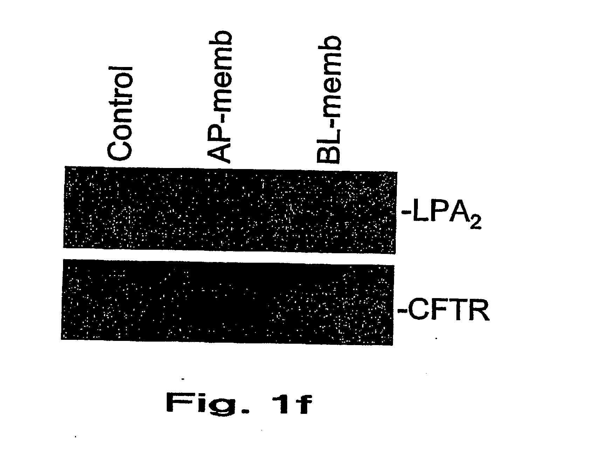

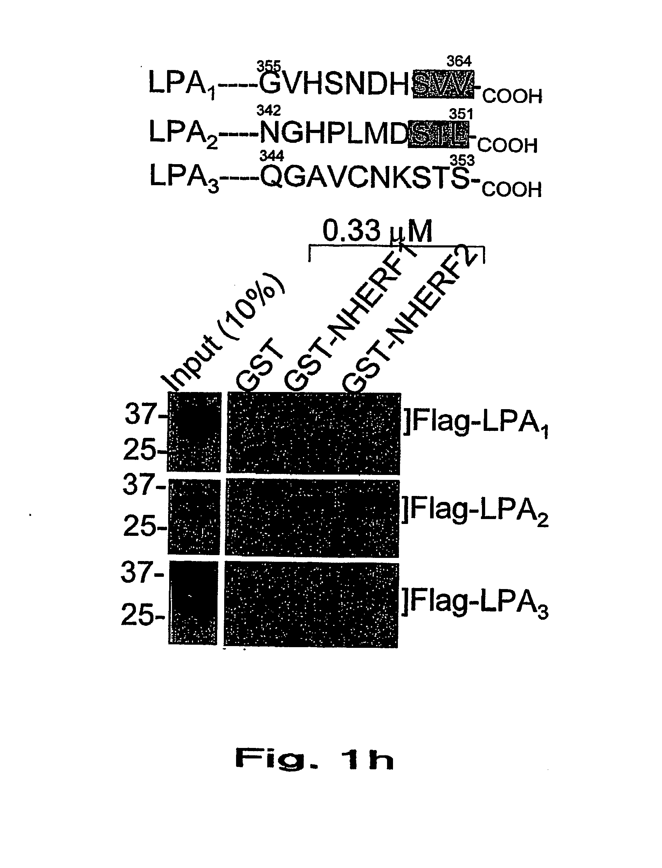

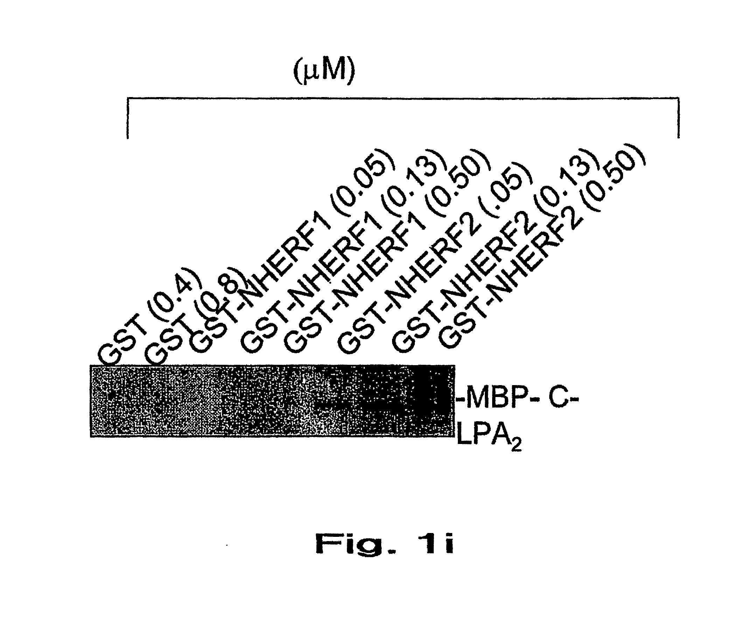

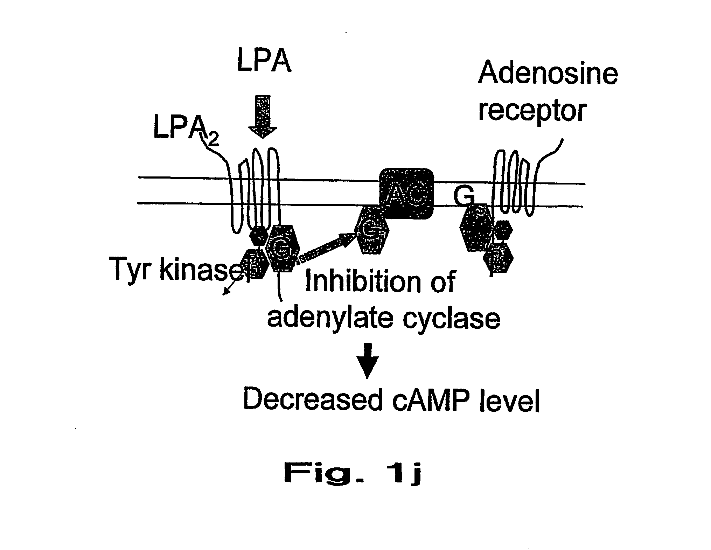

LPA₁ and LPA₂, but not LPA₃ transcripts, were present in mouse intestinal tissue (Fig. 1, A and B), and using human sequence-specific oligos for LPA₂, HT29-CL19A (colonic epithelial cells) and Calu-3 (serous gland epithelial cells) were also found to express LPA₂ (Fig. 1C). Using an LPA₂-specific antibody (Fig. 1D, left), the expression of LPA₂ in plasma membranes prepared from HT29-CL19A and Calu-3 cells (*13*) was verified. LPA₂ appeared as a major immunoreactive band of~50 kD (Fig. 1D, right).

LPA₂ was next colocalized in part to the apical surface of HT29-CL19A (Fig. 1E, top) using fluorescence confocal microscopy (*14*). Apical localization of LPA₂ was also confirmed using surface biotinylation (Fig. 1F, top) (*13*). The LPA₂ interacting partner NHERF2 (15) was expressed at various levels in epithelial cells (HT29-CL19A, Calu-3, etc.) and appeared as a ~50-kD immunoreactive band (Fig. 1G, upper right). NHERF2 was also localized to the apical surfaces in HT29-CL19A cells (Fig. 1G, bottom) where CFTR and LPA₂ are shown to reside (Fig. 1, E and F).

### Example 2 - LPA₂ Receptor Interaction with NHERF2 and CFTR

The C-terminal tails of LPA₁ and LPA₂, but not LPA₃, contain a consensus for the PDZ (PSD95 /Dlg /ZO-1) motif, the short COOH-terminal protein sequences that specifically interact with the PDZ-binding domains (usually ~70-90 amino acid sequences) of PDZ proteins (*16*) (Fig. 1H, upper, boxed area). Thus, LPA₁ and LPA₂ are likely candidates for PDZ-domain containing proteins to interact and regulate the receptor function. Pull-down assay demonstrated that LPA₂ bound NHERF2 with the highest affinity, whereas LPA₁ bound with rather weak affinity (Fig. 1H) (*15*). A direct interaction between the C-tail of LPA₂ and NHERF2 was also demonstrated (Fig. 1I).

Although all three LPA receptors (LPA_{1,} LPA₂, and LPA₃) respond to LPA, LPA₂ has the highest affinity to the lipid (*17*), leading to the activation of at least three distinct G-protein pathways: Gᵢ, Gq, and G_{12/13} (*6*, *7*). Activation of the receptors by LPA results in inhibition of the adenylate cyclase pathway, which in turn decreases cAMP levels (*5*-*7*). The Gᵢ activation pathway of the receptor is summarized in Fig. 1J. Given that LPA₂ binds NHERF2 (Fig. 1, H and I) and our previous demonstration that NHERF2 can form a macromolecular complex with CFTR (*18*), we hypothesized that LPA₂ might regulate CFTR Cl⁻ channel function. To test this, the effect of LPA on CFTR was monitored using iodide efflux measurements (*19*). LPA pretreatment reduced the response of Calu-3 cells to cpt-cAMP stimulation, while LPA alone had no effect on CFTR channel function (Fig. 2A). These results clearly demonstrate that LPA can specifically inhibit CFTR function in epithelial cells.

### Example 3 - LPA Inhibition of CFTR Function is Receptor-Mediated

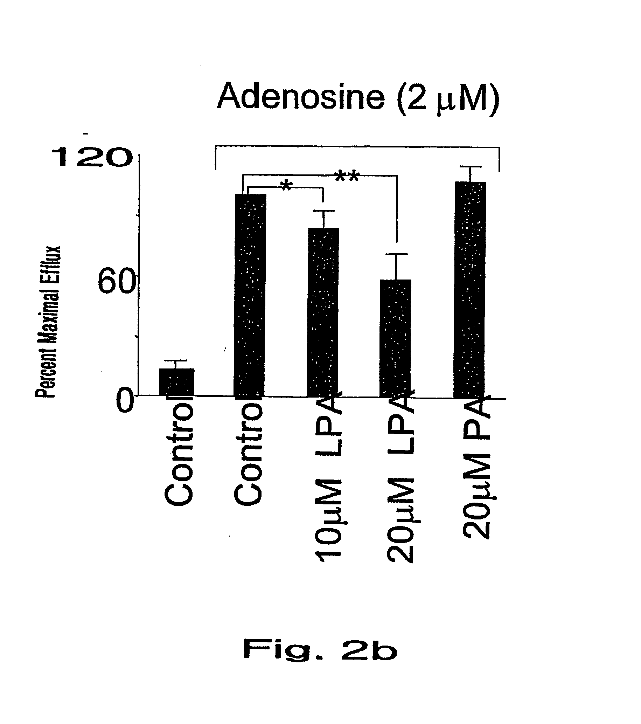

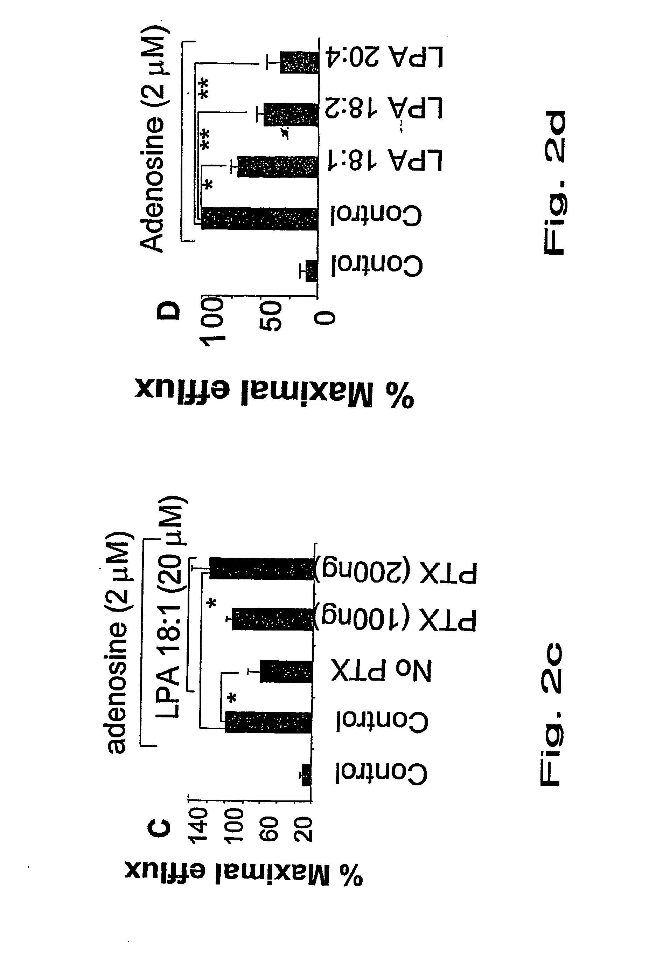

To test the hypothesis that the inhibition of CFTR function by LPA is receptor-mediated, Calu-3 cells were pretreated with pertussis toxin (PTX), which has been demonstrated to catalyze the ADP-ribosylation of the G_{αi} subunit, thus specifically disrupting the Gᵢ pathway (21). PTX pretreatment reversed LPA-dependent inhibition of CFTR function in a dose-dependent fashion (Fig. 2B). It is therefore likely that inhibition of CFTR function by LPA is receptor-mediated through Gᵢ pathway. A dose-dependent inhibition of CFTR activity by LPA was also observed when adenosine was used as CFTR agonist (Fig. 2C). In contrast, the related lipid, phosphatidic acid (PA), which is not a ligand for LPA receptors (*5*), did not show any significant effect on CFTR function (Fig. 2C). Several fatty acid analogs of LPA were screened, and the rank order of inhibition was LPA 20:4 > LPA 18:2 > LPA 18:1 (Fig. 2D), which matches the relative abundance of these analogs in human serum (22).

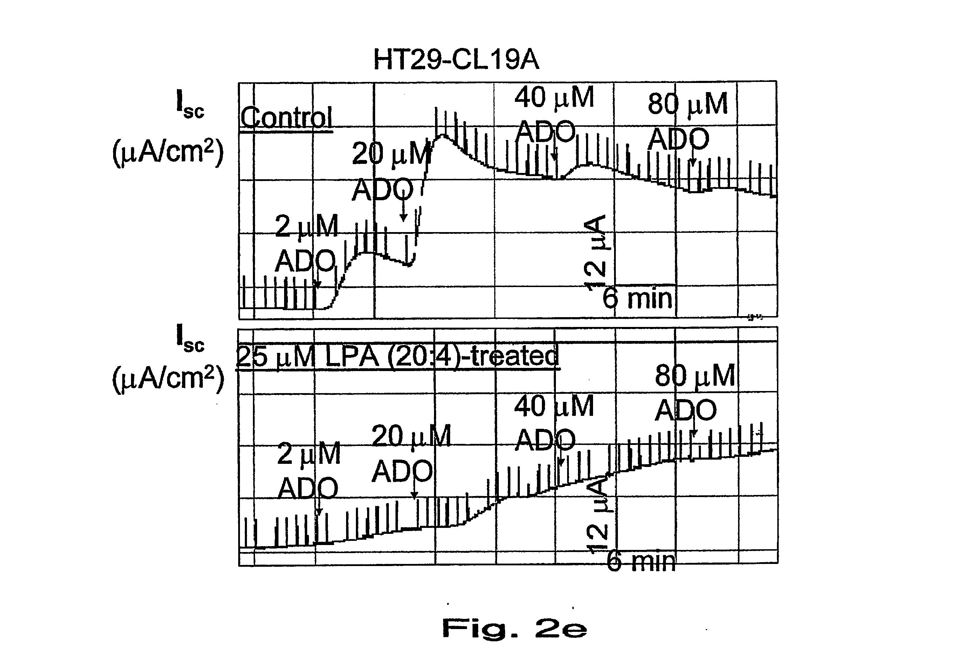

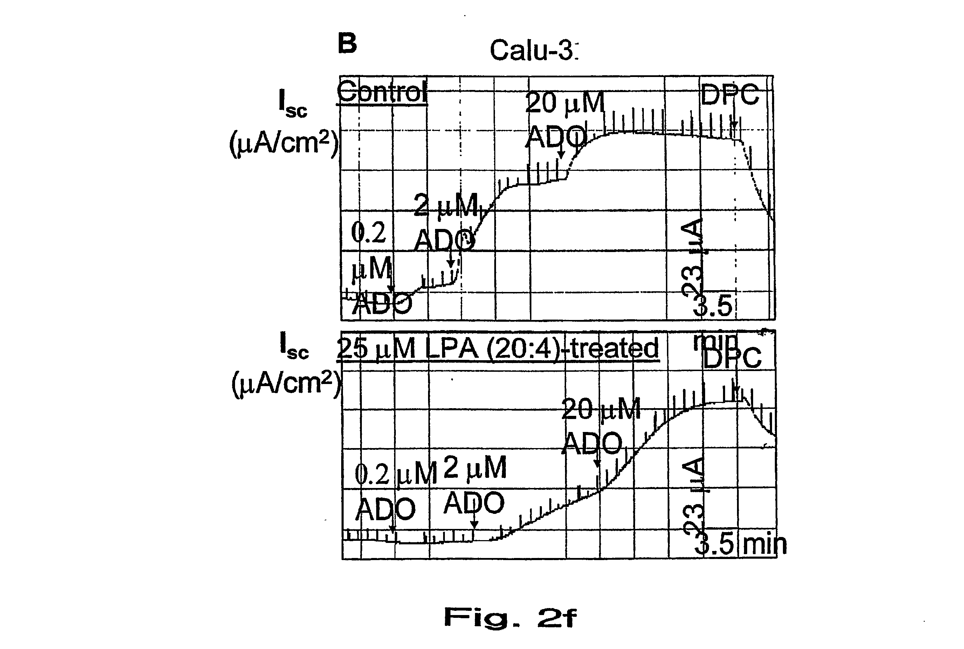

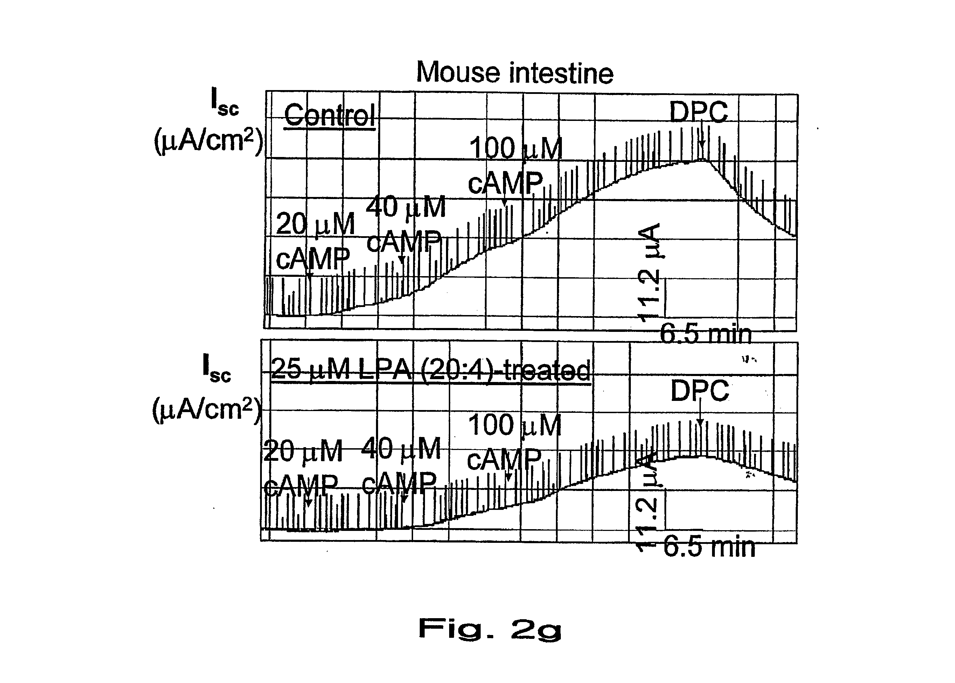

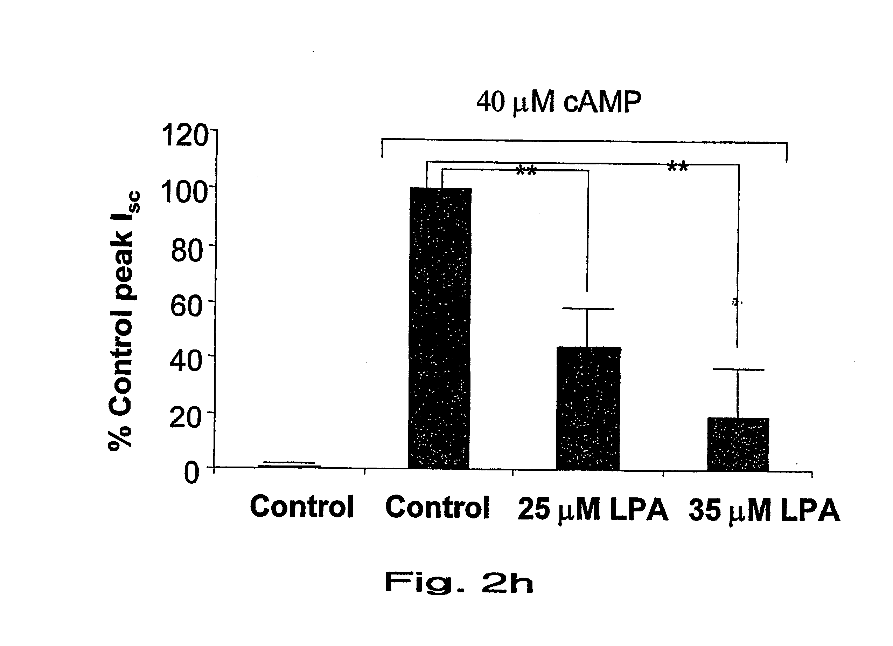

The effect of LPA on CFTR-dependent short-circuit currents (I_{sc}) was monitored on polarized epithelial monolayers (18). LPA significantly inhibited CFTR-dependent Cl⁻ currents in response to adenosine activation in HT29 cells (Fig. 2E), as well as Calu-3 cells (Fig. 2F). I_{sc} measured in response to cpt-cAMP stimulation was inhibited in LPA-pretreated isolated intestine tissue (Fig. 2G, bottom) in a dose-dependent fashion (Fig. 2H).

### Example 4 - LPA Inhibition of CTX-Induced Diarrhea

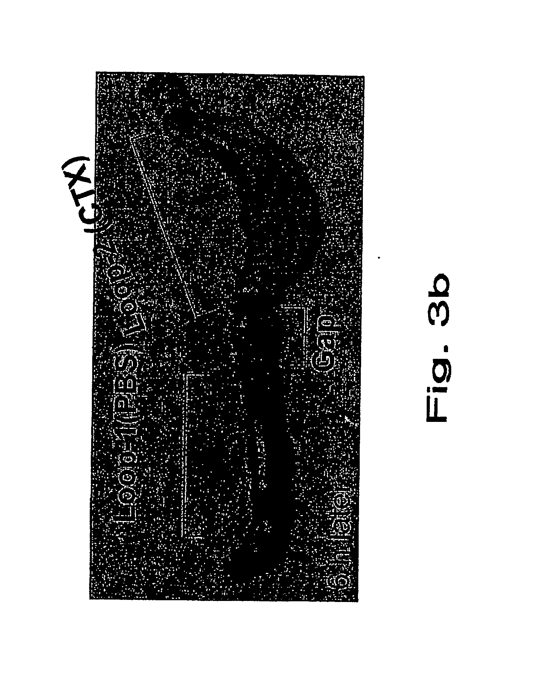

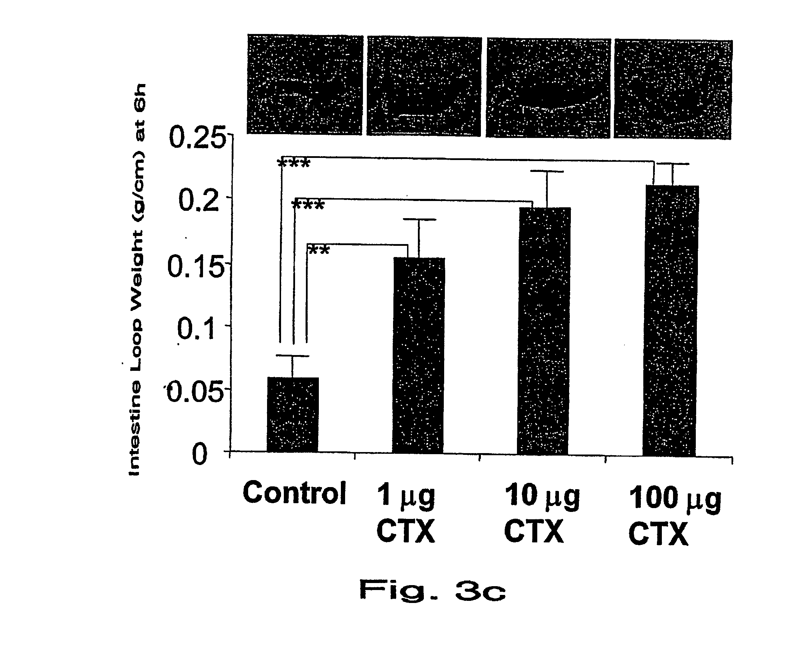

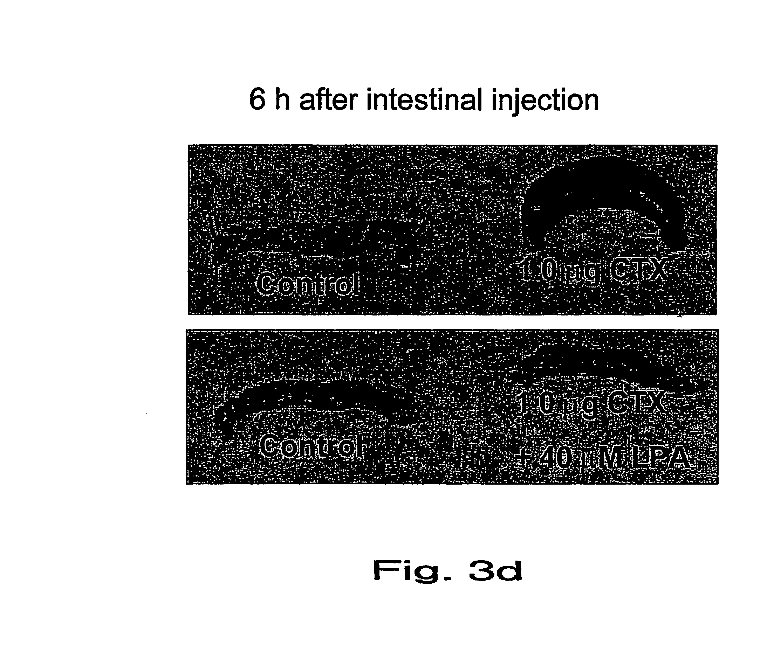

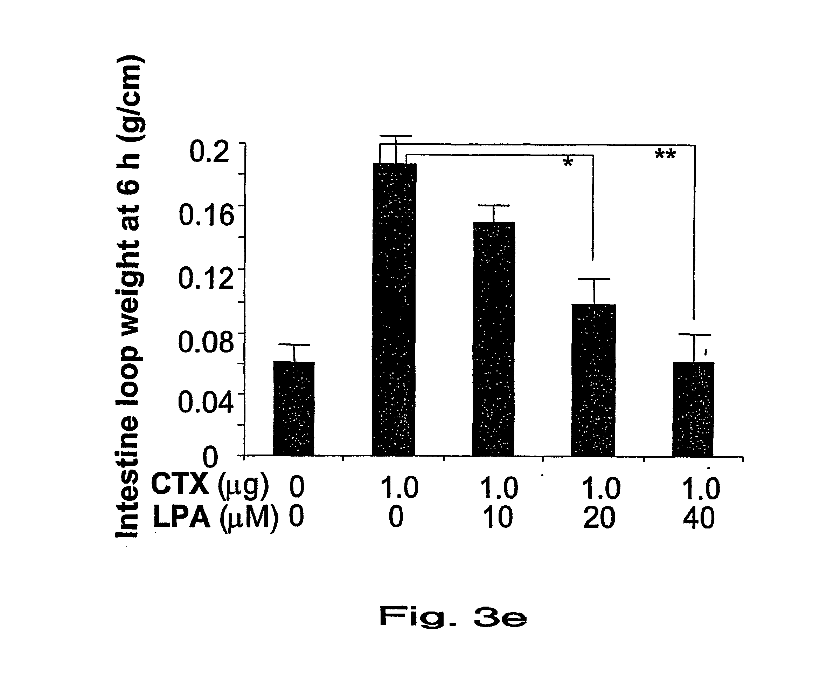

To provide proof of concept that LPA could be used in a therapeutic setting, the effect of LPA was tested on cholera toxin (CTX)-induced diarrhea model in mice (Fig. 3A) (23). There was a marked fluid accumulation in the CTX-treated ileal loop (Fig. 3B) in a dose-dependent manner (Fig. 3C), whereas the adjacent control loop (PBS alone) did not show any accumulation of fluid. LPA treatment efficiently reduced the fluid accumulation in the toxin-treated intestinal loops (Fig. 3D, bottom), suggesting that LPA has a significant inhibitory effect on CTX-mediated intestinal fluid secretion. Our results indicated a dose-dependent inhibition of LPA on CTX-elicited CFTR-dependent intestinal fluid secretion with 40 µM LPA significantly reducing fluid accumulation to the level of PBS control loops (Fig. 3E).

### Example 5 - LPA₂-NHERF2-CFTR Macromolecular Complex Is Required for LPA₂-Mediated Inhibition of the Chloride Channel

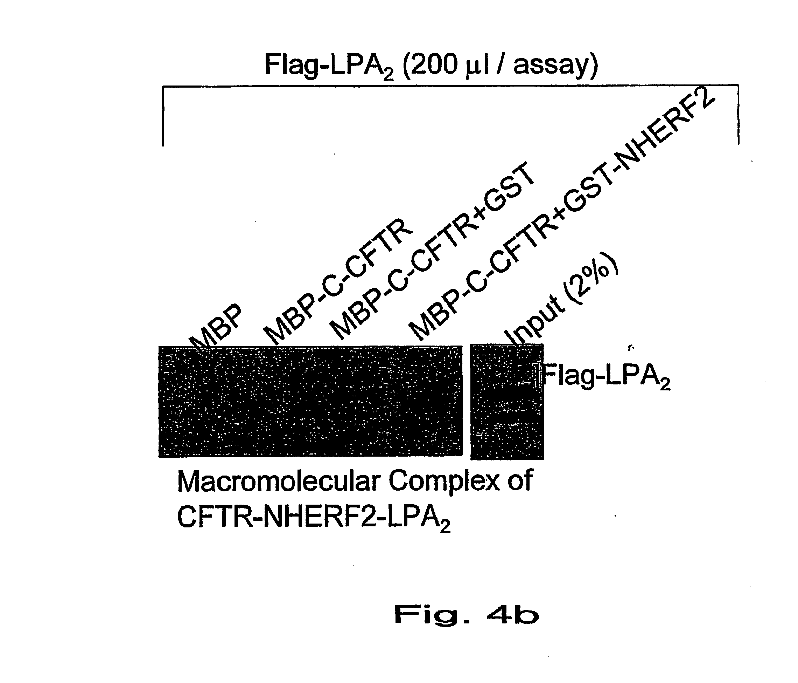

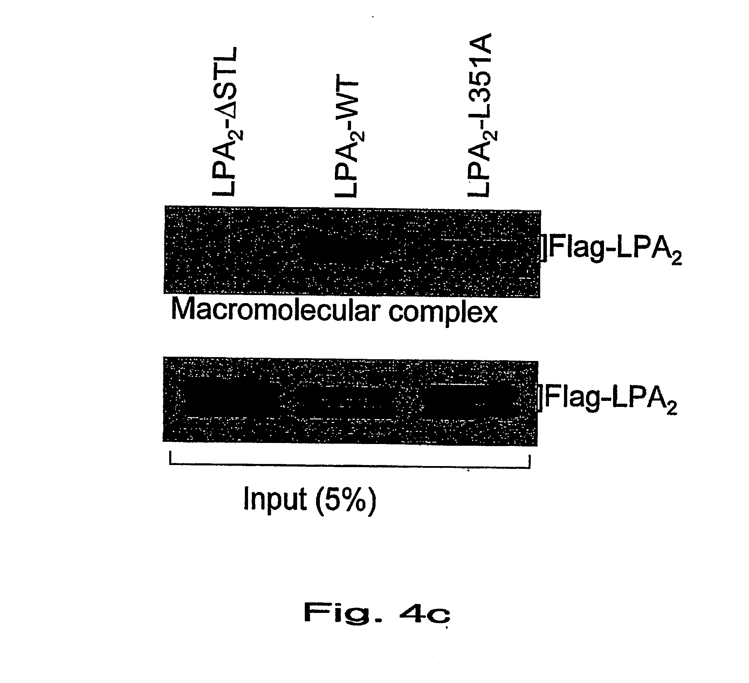

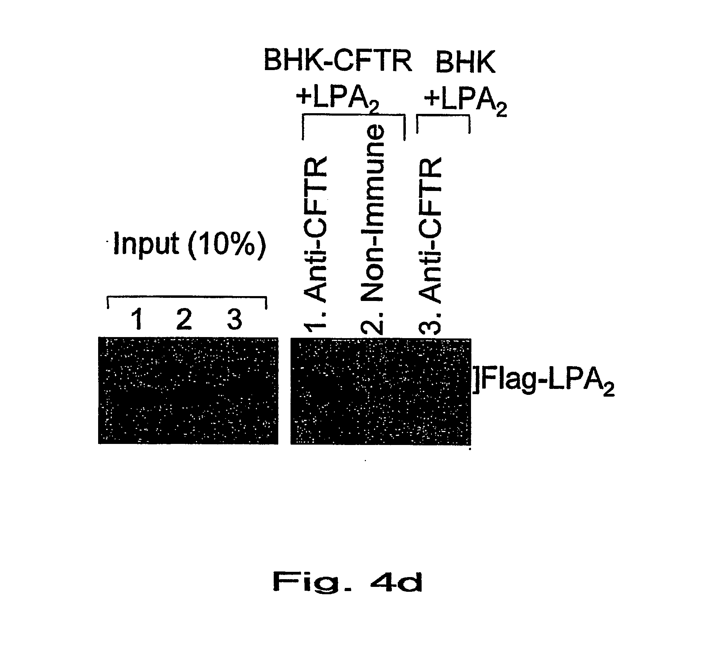

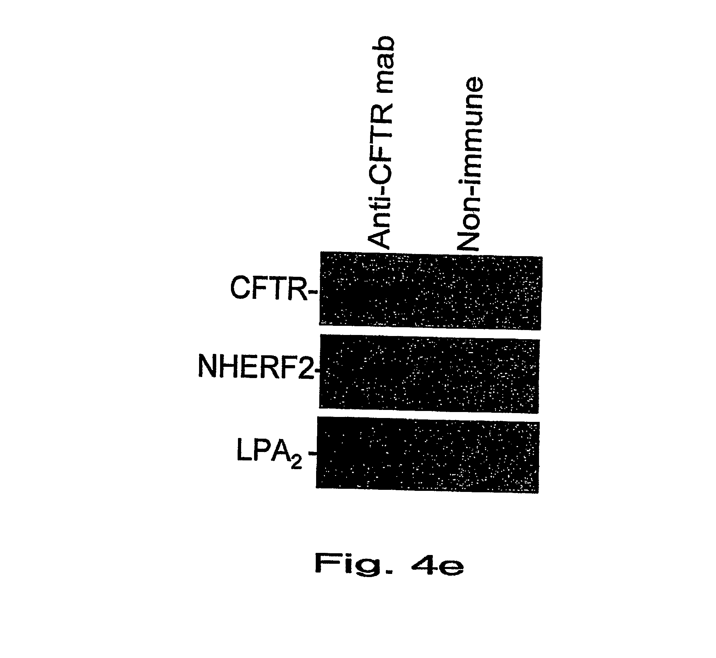

The inventors hypothesized that the interaction between CFTR and LPA₂ was likely mediated by NHERF2. To test this, we assembled a macromolecular complex of LPA₂, NHERF2, and CFTR *in vitro* (Fig. 4A) (*13*)*.* A macromolecular complex formed between MBP-C-CFTR, GST-NHERF2, and LPA₂ (Fig. 4B). MBP-C-CFTR did not bind directly to LPA₂ nor did the complex form in the presence of GST or MBP alone (Fig. 4B). The complex formation was PDZ-motif dependent. Mutating the last amino acid of LPA₂ (L351A) reduced the complex formation, whereas deleting the last three amino acids of LPA₂ (ΔSTL) completely eliminated the macromolecular complex formation (Fig. 4C). The *in vitro* macromolecular complex assembly does not indicate whether the complex exists in native membranes; therefore, cross-linking experiments were performed to capture the complex in over-expressing cells (*18*). Figure 4D shows a cross-linked complex containing CFTR and LPA₂ in BHK cells that express endogenous NHERF2. We next immunoprecipitated CFTR from Calu-3 cells and demonstrated that LPA₂ and NHERF2 were present in the complex (Fig. 4E). These studies suggest that a macromolecular complex of LPA₂-NHERF2-CFTR is likely present in the apical surface of epithelial cells.

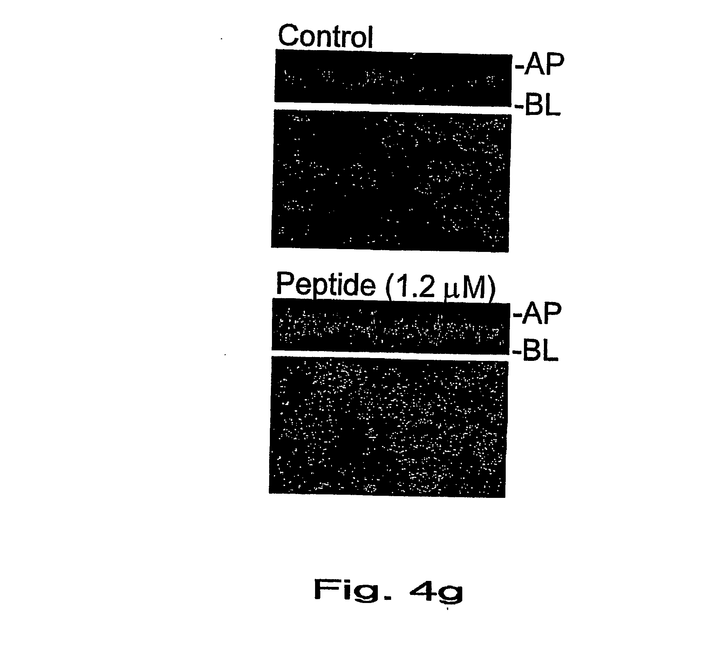

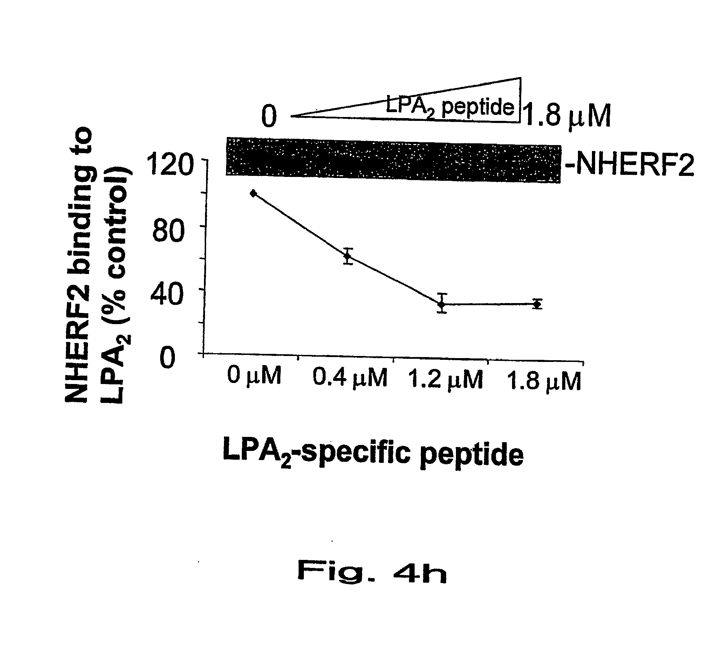

We next determined if a physical association of LPA₂ and CFTR via NHERF2 was essential for the LPA₂-mediated inhibition of the Cl⁻ channel. To disrupt the complex, we delivered an LPA₂ isoform-specific peptide containing the PDZ motif using the Chariot system (24) into polarized epithelial cells before short-circuit measurements. I_{sc} in response to adenosine (0-20 µM) in the presence of LPA was monitored. LPA₂-specific peptide significantly reversed the LPA-elicited inhibition on CFTR-dependent Cl⁻ currents (Fig. 4F, bottom). At the end of the experiment, the epithelial cells were fixed and immunostained. We observed higher intensity of fluorescence in the cells delivered with the peptide (Fig. 4G, bottom). We confirmed that the LPA₂ peptide could compete with LPA₂ for binding to NHERF2 in a dose-dependent manner (Fig. 4H).

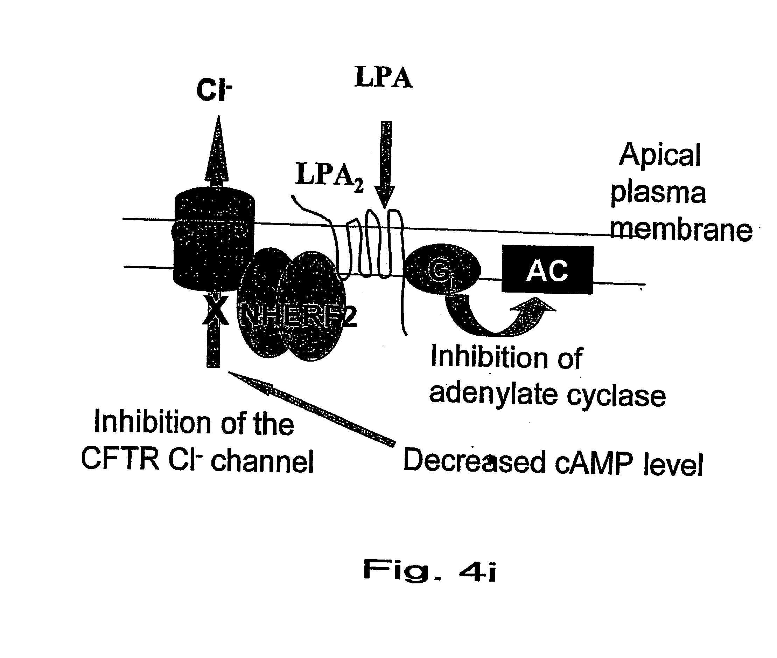

Given that LPA₂ receptors are expressed in the gut (Fig. 1, A to D), are localized to the apical surface (Fig. 1, E and F), and activate the Gᵢ pathway leading to decreased cAMP accumulation (Fig. 1J), we propose a novel model (Fig. 4I). According to our hypothesis, LPA₂ and CFTR are physically associated with NHERF2, which clusters LPA₂ and CFTR into a macromolecular complex at the apical plasma membrane of epithelial cells. This macromolecular complex is the foundation of functional coupling between LPA₂ signaling and CFTR-mediated Cl⁻ efflux. The model predicts that if the physical association is disrupted, functional activity will be compromised as well. Upon LPA stimulation of the receptor, adenylate cyclase is inhibited through the Gᵢ pathway, leading to a decrease in cAMP level. This decreased local or compartmentalized concentration of cAMP results in the reduced Cl⁻ channel activation in response to CFTR agonists (e.g., ADO). Our findings not only shed light on the detailed mechanism underlying the transduction pathway of the LPA₂ agonists, but also help clarify how LPA inhibits CFTR-dependent Cl⁻ channel activity, all of which will lead to alleviating diarrhea symptoms. Because LPA is richly available in certain forms of foods (3, 4), our proof of concept study might pave the way for the use of certain diets to control diarrhea.

### Example 6 - LPA₂ Agonist Inhibits CTX-Induced Chloride Channel Secretion

An open-loop mouse model was used in determining the effect of fatty alcohol thiophosphate 18:1d9 (FAP) on cholera toxin induced Cl- secretion. The animal protocol was reviewed and approved by the Animal Care and Use Committee at UTHSC. Male C57BL/6 mice aged around 8 weeks were purchased from Harlan (Indianapolis, IN) and maintained on a 12:12-h light-dark cycle and fed standard laboratory mouse chow and water ad libitum. After 2 weeks of acclimatization, mice were fasted with free access to water for 24 hours prior to the open-loop experiment. Mice were lavaged with 100 µl 7% NaHCO₃ buffer containing 10 µg/ml CTX in the presence or absence of 100 µM LPA (18:1) or 100 µm FAP using an orogastric feeding needle, and sacrificed 6 hours later. The entire small intestine from pylorus to cecum was exteriorized and stripped off mesenteric and connective tissue, and then weighed. The small intestine was weighed again after flushing of its content. Intestine weight ratio and intestinal fluid accumulation were calculated as described by Werkman et al. (*20*). A ratio of intestinal weight before to intestinal weight after luminal fluid removal was used to determine the Cl-secretion into the intestinal lumem. As shown in Figure 5, both LPA and FAP 18:1d9 treatments resulted in a statistically significant reduction in the ratio. As shown in Figure 6, FAP 18:1d9 significantly reduced intestinal fluid accumulation (right panel).

### List of Cited References

1. M. J. Welsh, L. C. Tsui, T. F. Boat, A. L. Beaudet, in The metabolic and molecular basis of inherited diseases: Membrane transport systems, C. R. Scriver, A. L. Beaudet, W. S. Sly, D. Valle, Eds. (McGraw-Hill, New York, 1995), vol. 3.
2. S. E. Gabriel, K. N. Brigman, B. H. Koller, R. C. Boucher, M. J. Stutts, Science 266, 107 (1995).
3. A. Tokumura et al., Lipids 13,468 (1978).
4. S. Nakane, A. Tokumura, K. Waku, T. Sugiura, Lipids 36, 413 (2001).
5. G. Tigyi, A. L. Parrill, Prog. Lipid Res. 42, 498 (2003).
6. G. B. Mills, W. H. Moolenaar, Nat. Rev. Cancer 3, 582 (2003).
7. I. Ishii, N. Fukushima, X. Ye, J. Chun, Annu. Rev. Biochem. 73, 321 (2004).
8. A. Sturm, A. U. Dignass, Biochim. Biophys. Acta. 1582, 282 (2002).
9. A. Sturm et al, Digestion 66, 23 (2002).
10. W. Deng et al., Gastroenterology 123, 206 (2002).
11. W. Deng, D. A. Wang, E. Gosmanova, L. R. Johnson, G. Tigyi, Am. J. Physiol. Gastrointest. Liver Physiol. 284, G821 (2003).
12. M. A. Funk-Archuleta, M. W. Foehr, L. D. Tomei, K. L. Hennebold, I. C. Bathurst, Nutr. Cancer 29, 217 (1997).
13. A. P. Naren et al., Proc. Natl. Acad. Sci. USA 100, 342 (2003).
14. P. Sheth, S. Basuroy, C. Li, A. P. Naren, R. K. Rao, J. Biol. Chem. 278, 49239 (2003).
15. Y. S. Oh et al., Mol. Cell. Biol. 24, 5069 (2004).
16. Z. Songyang et al., Science 275, 73 (1997).
17. D.-S. Im et al., Mol. Pharmacol. 57, 753 (2000).
18. C. Li, K. Roy, K. Dandridge, A. P. Naren, J. Biol. Chem. 279, 24673 (2004).
19. X.-B. Chang et al., J. Biol. Chem. 268, 11304 (1993).
20. Werkman et al.
21. G. M. Bokoch, T. Katada, J. K. Northup, E. L. Hewlett, A. G. Gilman, J. Biol. Chem. 25,2072 (1983).
22. T. Sano et al., J. Biol. Chem. 277, 21197 (2002).
23. T. Ma et al., J. Clin. Invest. 110, 1651 (2002).
24. M. C. Morris, J. Depollier, J. Mery, F. Heitz, G. Divita, Nat. Biotechnol. 19, 1173 (2001).
25. A. P. Naren, Methods Mol. Med. 70, 175 (2002).
26. C. Li, K. Roy, K. Dandridge, A. P. Naren, J. Biol. Chem. 279,24673 (2004).
27. A. P. Naren, M. W. Quick, J. F. Collawn, D. J. Nelson, K. L. Kirk, Proc. Natl. Acad. Sci. U. S. A 95,10972 (1998).
28. A. P. Naren, Methods Mol. Med. 70, 175 (2002).
29. G. Tigyi, A. L. Parrill, Prog. Lipid Res. 42, 498 (2003).
30. X.-B. Chang et al., J. Biol. Chem. 268, 11304 (1993).
31. J. J. Smith, P. H. Karp, M. J. Welsh, (1994) J. Clin. Invest. 93, 307-311
32. L. L. Clarke, M. C. Harline, Am. J. Physiol. Gastrointest. Liver Physiol. 274, G718 (1998).
33. T. Ma et al., J. Clin. Invest. 110, 1651 (2002).
34. A. P. Naren et al., Proc. Natl. Acad. Sci. U S A 100, 342 (2003).
35. M. C. Morris, J. Depollier, J. Mery, F. Heitz, G. Divita, Nat. Biotechnol. 19, 1173 (2001).

## Claims

1. A composition comprising one or more LPA₂ receptor agonists for use in a method of treating or preventing diarrhea wherein the one or more LPA₂ receptor agonists are selected from the group consisting of (*R*)-octanoic acid 1-octanoyloxymethyl-2-thiophosphonooxy-ethyl ester, (*S*)-octanoic acid 1-octanoyloxymethyl-2-thiophosphonooxy-ethyl ester, (*R*)-thiophosphoric acid O-(2,3-bis-octyloxy-propyl) ester, mono potassium thiophosphoric acid O-(2-heptadec-9-enyl-(1,3)-dioxolan-4-ylmethyl) ester, mono potassium phosphoric acid O-(2-heptadec-9,12,15-trienyl-(1,3)- dioxolan-4-ylmethyl) ester, (2R,4S) mono potassium thiophosphoric acid O-(2-heptadec-9-enyl-(1,3)-dioxolan-4-ylmethyl) ester, (2S,4S) mono potassium thiophosphoric acid O-(2-heptadec-9-enyl-(1,3)-dioxolan-4-ylmethyl) ester, (2R,4R) mono potassium thiophosphoric acid O-(2-heptadec-9-enyl-(1,3)-dioxolan-4-ylmethyl) ester, (2R,4R) mono potassium phosphoric acid O-(2-heptadec-9-enyl-(1,3)-dioxolan-4-ylmethyl) ester, (2S,4R) mono potassium thiophosphoric acid O-(2-heptadec-9-enyl-(1,3)-dioxolan-4-ylmethyl) ester, and combinations thereof.

2. The composition for use in a method of treating or preventing diarrhea according to claim 1 wherein the composition further comprises an aqueous carrier.

3. The composition for use in a method of treating or preventing diarrhea according to claim 2 wherein the carrier includes one or more electrolytes.

4. The composition for use in a method of treating or preventing diarrhea according to claim 1 **characterized in that** it is administered orally.

5. The composition for use in a method of treating or preventing diarrhea according to claim 1 **characterized in that** it is administered intravenously.

6. The composition for use in a method of treating or preventing diarrhea according to claim 1 wherein said method comprises administration of said composition prior to onset of diarrhea symptoms.

7. The composition for use in a method of treating or preventing diarrhea according to claim 1 wherein said method comprises administration of said composition after onset of diarrhea symptoms.

8. The composition for use in a method of treating or preventing diarrhea according to claim 1 wherein the diarrhea is induced by a bacterial infection.

9. The composition for use in a method of treating or preventing diarrhea according to claim 8 wherein the bacterial infection is caused by *Vibrio cholerae, Escherichia coli*, or *Clostridium difficile.*

10. The composition for use in a method of treating or preventing diarrhea according to claim 1 wherein the diarrhea is induced by an immunoinflammatory response.

11. The composition for use in a method of treating or preventing diarrhea according to claim 1 wherein the composition comprises a food or nutritional supplement containing an effective amount of LPA (lysophosphatidic acid).

12. A composition comprising:
an aqueous carrier,
one or more LPA₂ receptor agonists; and
one or more electrolytes.
wherein the one or more LPA₂ receptor agonists are selected from (R)-octanoic acid 1-octanoyloxymethyl-2-thiophosphonooxy-ethyl ester, (S)-octanoic acid 1-octanoyloxymethyl-2-thiophosphonooxy-ethyl ester, (R)-thiophosphoric acid O-(2,3-bis-octyloxy-propyl) ester, mono potassium thiophosphoric acid O-(2-heptadec-9-enyl-(1,3)-dioxolan-4-ylmethyl) ester, mono potassium phosphoric acid O-(2-heptadec-9,12,15-trienyl-(1,3)-dioxolan-4-ylmethyl) ester, (2R,4S) mono potassium thiophosphoric acid O-(2-heptadec-9-enyl-(1,3)-dioxolan-4-ylmethyl) ester, (2S,4S) mono potassium thiophosphoric acid O-(2-heptadec-9-enyl-(1,3)-dioxolan-4-ylmethyl) ester, (2R,4R) mono potassium thiophosphoric acid O-(2-heptadec-9-enyl-(1,3)-dioxolan-4-ylmethyl) ester, (2R,4R) mono potassium phosphoric acid O-(2-heptadec-9-enyl-(1,3)-dioxolan-4-ylmethyl) ester, (2S,4R) mono potassium thiophosphoric acid O-(2-heptadec-9-enyl-(1,3)- dioxolan-4-ylmethyl) ester, and combinations thereof.

13. The composition of claim 12 wherein the aqueous carrier and one or more electrolytes comprise a saline solution.

14. The composition of claim 12 wherein the aqueous carrier and one or more electrolytes comprise Ringer's lactate.

15. The composition of claim 12 further comprising at least one antibiotic.

## Patentansprüche

1. Zusammensetzung, die einen oder mehrere LPA₂-Rezeptor-Agonisten umfasst, zur Verwendung in einem Verfahren zur Behandlung oder Prävention von Diarrhöe, wobei der eine oder die mehreren LPA₂-Rezeptor-Agonisten aus der Gruppe bestehend aus (*R*)-Octansäure-1-octanoyloxymethyl-2-thiophosphonooxyethylester, (*S*)-Octansäure-1-octanoyloxymethyl-2-thiophosphonooxyethylester, (*R*)-Thiophosphorsäure-O-(2,3-bisoctyloxypropyl)ester, Thiophosphorsäure-O-(2-heptadec-9-enyl-(1,3)-dioxolan-4-ylmethyl)ester-Monokalium, Phosphorsäure-O-(2-heptadec-9,12,15-trienyl-(1,3)-dioxolan-4-ylmethyl)ester-Monokalium, (2R,4S)-Thiophosphorsäure-O-(2-heptadec-9-enyl-(1,3)-dioxolan-4-ylmethyl)ester-Monokalium, (2S,4S)-Thiophosphorsäure-O-(2-heptadec-9-enyl-(1,3)-dioxolan-4-ylmethyl)ester-Monokalium, (2R,4R)-Thiophosphorsäure-O-(2-heptadec-9-enyl-(1,3)-dioxolan-4-ylmethyl)ester-Monokalium, (2R,4R)-Phosphorsäure-O-(2-heptadec-9-enyl-(1,3)-dioxolan-4-ylmethyl)ester-Monokalium, (2S,4R)-Thiophosphorsäure-O-(2-heptadec-9-enyl-(1,3)-dioxolan-4-ylmethyl)ester-Monokalium und Kombinationen davon ausgewählt sind.

2. Zusammensetzung zur Verwendung in einem Verfahren zur Behandlung oder Prävention von Diarrhöe nach Anspruch 1, wobei die Zusammensetzung weiterhin einen wässrigen Trägerstoff umfasst.

3. Zusammensetzung zur Verwendung in einem Verfahren zur Behandlung oder Prävention von Diarrhöe nach Anspruch 2, wobei der Trägerstoff einen oder mehrere Elektrolyten beinhaltet.

4. Zusammensetzung zur Verwendung in einem Verfahren zur Behandlung oder Prävention von Diarrhöe nach Anspruch 1, **dadurch gekennzeichnet, dass** sie oral verabreicht wird.

5. Zusammensetzung zur Verwendung in einem Verfahren zur Behandlung oder Prävention von Diarrhöe nach Anspruch 1, **dadurch gekennzeichnet, dass** sie intravenös verabreicht wird.

6. Zusammensetzung zur Verwendung in einem Verfahren zur Behandlung oder Prävention von Diarrhöe nach Anspruch 1, wobei das Verfahren die Verabreichung der Zusammensetzung vor dem Einsetzen von Diarrhöesymptomen umfasst.

7. Zusammensetzung zur Verwendung in einem Verfahren zur Behandlung oder Prävention von Diarrhöe nach Anspruch 1, wobei das Verfahren die Verabreichung der Zusammensetzung nach dem Einsetzen von Diarrhöesymptomen umfasst.

8. Zusammensetzung zur Verwendung in einem Verfahren zur Behandlung oder Prävention von Diarrhöe nach Anspruch 1, wobei die Diarrhöe von einer bakteriellen Infektion bewirkt wird.

9. Zusammensetzung zur Verwendung in einem Verfahren zur Behandlung oder Prävention von Diarrhöe nach Anspruch 8, wobei die bakterielle Infektion von *Vibrio cholerae, Escherichia coli* oder *Clostridium difficile* verursacht wird.

10. Zusammensetzung zur Verwendung in einem Verfahren zur Behandlung oder Prävention von Diarrhöe nach Anspruch 1, wobei die Diarrhöe von einer immunologischen Entzündungsreaktion bewirkt wird.

11. Zusammensetzung zur Verwendung in einem Verfahren zur Behandlung oder Prävention von Diarrhöe nach Anspruch 1, wobei die Zusammensetzung ein Nahrungsmittel oder Nahrungsergänzungsmittel umfasst, das eine wirksame Menge von LPA (Lysophosphatidsäure) enthält.

12. Zusammensetzung, die Folgendes umfasst:
einen wässrigen Trägerstoff;
einen oder mehrere LPA₂-Rezeptor-Agonisten und
einen oder mehrere Elektrolyten,
wobei der eine oder die mehreren LPA₂-Rezeptor-Agonisten aus (R)-Octansäure-1-octanoyloxymethyl-2-thiophosphonooxyethylester, (*S*)-Octansäure-1-octanoyloxymethyl-2-thiophosphonooxyethylester, (R)-Thiophosphorsäure-O-(2,3-bisoctyloxypropyl)ester, Thiophosphorsäure-O-(2-heptadec-9-enyl-(1,3)-dioxolan-4-ylmethyl)ester-Monokalium, Phosphorsäure-O-(2-heptadec-9,12,15-trienyl-(1,3)-dioxolan-4-ylmethyl)ester-Monokalium, (2R,4S)-Thiophosphorsäure-O-(2-heptadec-9-enyl-(1,3)-dioxolan-4-ylmethyl)ester-Monokalium, (2S,4S)-Thiophosphorsäure-O-(2-heptadec-9-enyl-(1,3)-dioxolan-4-ylmethyl)ester-Monokalium, (2R,4R)-Thiophosphorsäure-O-(2-heptadec-9-enyl-(1,3)-dioxolan-4-ylmethyl)ester-Monokalium, (2R,4R)-Phosphorsäure-O-(2-heptadec-9-enyl-(1,3)-dioxolan-4-ylmethyl)ester-Monokalium, (2S,4R)-Thiophosphorsäure-O-(2-heptadec-9-enyl-(1,3)-dioxolan-4-ylmethyl)ester-Monokalium und Kombinationen davon ausgewählt sind.

13. Zusammensetzung nach Anspruch 12, wobei der wässrige Trägerstoff und der eine oder die mehreren Elektrolyten eine Kochsalzlösung umfassen.

14. Zusammensetzung nach Anspruch 12, wobei der wässrige Trägerstoff und der eine oder die mehreren Elektrolyten eine Ringer-Laktatlösung umfassen.

15. Zusammensetzung nach Anspruch 12, die weiterhin mindestens ein Antibiotikum umfasst.

## Revendications

1. Composition comprenant un ou plusieurs agonistes du récepteur LPA₂ pour une utilisation dans un procédé de traitement ou de prévention de la diarrhée, où les un ou plusieurs agonistes du récepteur LPA₂ sont choisis dans le groupe constitué par l'ester 1-octanoyloxyméthyl-2-thiophosphonooxy-éthylique de l'acide (*R*)-octanoïque, l'ester 1-octanoyloxyméthyl-2-thiophosphonooxy-éthylique de l'acide (*S*)-octanoïque, l'ester O-(2,3-bis-octyloxy-propylique) de l'acide (R)-thiophosphorique, l'ester O-(2-heptadéc-9-ényl-(1,3)-dioxolan-4-ylméthylique) de l'acide (R)-thiophosphorique monopotassique, l'ester O-(2-heptadéc-9,12,15-triényl-(1,3)-dioxolan-4-ylméthylique) de l'acide phosphorique monopotassique, l'ester 0-(2-heptadéc-9-ényl-(1,3)-dioxolan-4-ylméthylique) de l'acide (2R,4S) thiophosphorique monopotassique, l'ester O-(2-heptadéc-9-ényl-(1,3)-dioxolan-4-ylméthylique) de l'acide (2S,4S) thiophosphorique monopotassique, l'ester 0-(2-heptadéc-9-ényl-(1,3)-dioxolan-4-ylméthylique) de l'acide (2R,4R) thiophosphorique monopotassique, l'ester O-(2-heptadéc-9-ényl-(1,3)-dioxolan-4-ylméthylique) de l'acide (2R,4R) phosphorique monopotassique, l'ester 0-(2-heptadéc-9-ényl-(1,3)-dioxolan-4-ylméthylique) de l'acide (2S,4R) phosphorique monopotassique, et des combinaisons de ceux-ci.

2. Composition pour une utilisation dans un procédé de traitement ou de prévention de la diarrhée selon la revendication 1, où la composition comprend en outre un support aqueux.

3. Composition pour une utilisation dans un procédé de traitement ou de prévention de la diarrhée selon la revendication 2, dans laquelle le support comprend un ou plusieurs électrolytes.

4. Composition pour une utilisation dans un procédé de traitement ou de prévention de la diarrhée selon la revendication 1, **caractérisée en ce qu'**elle est administrée par voie orale.

5. Composition pour une utilisation dans un procédé de traitement ou de prévention de la diarrhée selon la revendication 1, **caractérisée en ce qu'**elle est administrée par voie intraveineuse.

6. Composition pour une utilisation dans un procédé de traitement ou de prévention de la diarrhée selon la revendication 1, où ledit procédé comprend l'administration de ladite composition avant l'installation des symptômes diarrhéiques.

7. Composition pour une utilisation dans un procédé de traitement ou de prévention de la diarrhée selon la revendication 1, où ledit procédé comprend l'administration de ladite composition après l'installation des symptômes diarrhéiques.

8. Composition pour une utilisation dans un procédé de traitement ou de prévention de la diarrhée selon la revendication 1, où la diarrhée est induite par une infection bactérienne.

9. Composition pour une utilisation dans un procédé de traitement ou de prévention de la diarrhée selon la revendication 8, où l'infection bactérienne est provoquée par *Vibrio cholerae, Escherichia coli* ou *Clostridium difficile.*

10. Composition pour une utilisation dans un procédé de traitement ou de prévention de la diarrhée selon la revendication 1, où la diarrhée est induite par une réponse immuno-inflammatoire.

11. Composition pour une utilisation dans un procédé de traitement ou de prévention de la diarrhée selon la revendication 1, où la composition comprend un aliment ou un complément nutritionnel contenant une quantité efficace de LPA (acide lysophosphatidique).

12. Composition comprenant :
un support aqueux,
un ou plusieurs agonistes du récepteur LPA₂ ; et
un ou plusieurs électrolytes,
dans laquelle les un ou plusieurs agonistes du récepteur LPA₂ sont choisis parmi l'ester 1-octanoyloxyméthyl-2-thiophosphonooxy-éthylique de l'acide (*R*)-octanoïque, l'ester 1-octanoyloxyméthyl-2-thiophosphonooxy-éthylique de l'acide (*S*)-octanoïque, l'ester O-(2,3-bis-octyloxy-propylique) de l'acide (R)-thiophosphorique, l'ester O-(2-heptadéc-9-ényl-(1,3)-dioxolan-4-ylméthylique) de l'acide (*R*)-thiophosphorique monopotassique, l'ester O-(2-heptadéc-9,12,15-triényl-(1,3)-dioxolan-4-ylméthylique) de l'acide phosphorique monopotassique, l'ester O-(2-heptadéc-9-ényl-(1,3)-dioxolan-4-ylméthylique) de l'acide (2R,4S) thiophosphorique monopotassique, l'ester O-(2-heptadéc-9-ényl-(1,3)-dioxolan-4-ylméthylique) de l'acide (2S,4S) thiophosphorique monopotassique, l'ester O-(2-heptadéc-9-ényl-(1,3)-dioxolan-4-ylméthylique) de l'acide (2R,4R) thiophosphorique monopotassique, l'ester O-(2-heptadéc-9-ényl-(1,3)-dioxolan-4-ylméthylique) de l'acide (2R,4R) phosphorique monopotassique, l'ester O-(2-heptadéc-9-ényl-(1,3)-dioxolan-4-ylméthylique) de l'acide (2S,4R) phosphorique monopotassique, et des combinaisons de ceux-ci.

13. Composition selon la revendication 12, dans laquelle le support aqueux et un ou plusieurs électrolytes comprennent une solution saline.

14. Composition selon la revendication 12, dans laquelle le support aqueux et un ou plusieurs électrolytes comprennent de la solution de Ringer lactate.

15. Composition selon la revendication 12, comprenant en outre au moins un antibiotique.
